# EUROPEAN PATENT APPLICATION

(11) **EP 1 437 352 A1**
(43) Date of publication of application: **14.07.2004**
(21) Application number: 02768002.4
(22) Date of filing: 24.09.2002
(51) Int. Cl.: C07D 401/12, C07D 405/14, A61K 31/4439, A61P 1/04, A61P 31/04, A61P 35/00, A61P 43/00

(54) **BENZIMIDAZOLE COMPOUND, PROCESS FOR PRODUCING THE SAME, AND USE THEREOF**

(30) Priority: 25.09.2001 JP 2001292619; 22.02.2002 JP 2002047204
(71) Applicant: Takeda Chemical Industries, Ltd., Osaka-shi, Osaka 541-0045 (JP)
(72) Inventor: KAMIYAMA, Keiji, Ibaraki-shi, Osaka 567-0037 (JP); SATO, Fumihiko, Suita-shi, Osaka 565-0872 (JP); BANNO, Hiroshi, Kawanishi-shi, Hyogo 666-0004 (JP); HASUOKA, Atsushi, Takatsuki-shi, Osaka 569-0043 (JP)
(74) Representative: Wright, Robert Gordon McRae
(86) International application number: PCT/JP2002/009746
(87) International publication number: WO 2003/027098

(57) **Abstract**

The present invention relates to a compound represented by the following formula 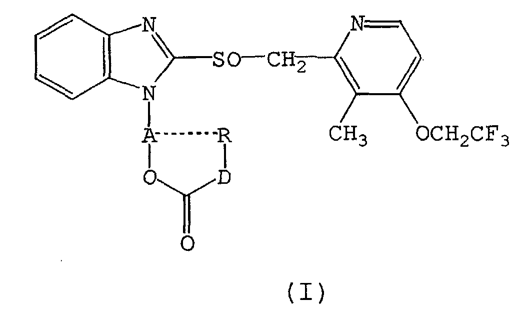 wherein each symbol is as defined in the specification, or a salt thereof, which has superior stability to acid and which is a prodrug of 2-[[[3-methyl-4-(2,2,2-trifluoroethoxy)-2-pyridyl]methyl]sulfinyl]-1H-benzimidazole. This compound (1) shows a superior anti-ulcer activity, a gastric acid secretion-suppressive action, a mucosa-protecting action, an anti-*Helicobacter pylori* action and the like in living organisms, (2) shows low toxicity, (3) shows superior stability to acid (which obviates the need to formulate an enteric-coated preparation, thereby lowering the cost, and reduces the size of preparation to facilitate swallowing for patients having difficulty in swallowing), (4) shows faster absorption than enteric-coated preparations (rapid expression of gastric acid secretion-suppressive action), and (5) is sustainable.

## Description

### Technical Field

The present invention relates to a benzimidazole compound, which is converted to a proton pump inhibitor in living organisms and shows an anti-ulcer activity and the like, a production method thereof and use thereof.

### Background Art

A proton pump inhibitor, 2-[[[3-methyl-4-(2,2,2-trifluoroethoxy)-2-pyridyl]methyl]sulfinyl]-1H-benzimidazole, and a salt thereof having an anti-ulcer activity are reported in JP-A-61-50978 and the like.

For oral administration, however, since the above-mentioned compounds are unstable to acids, they are formulated into an enteric-coated preparation, filled in a capsule and administered to prevent decomposition by gastric acid. Therefore, the development of a prodrug of the above-mentioned compound, which is stable to acid and resists decomposition by gastric acid, has been desired, and such prodrug is reported in US Patent No. 6,093,734. In addition, prodrugs of proton pump inhibitors other than the above-mentioned prodrugs have been disclosed in US Patent Nos. 4,045,563, 4,686,230, 4,873,337, 4,965,269, 5,021,433 and the like.

In view of the above situation, the development of a prodrug of a proton pump inhibitor having superior stability to acid has been desired.

It is therefore an object of the present invention to provide a benzimidazole compound having superior stability to acid, which is converted to 2-[[[3-methyl-4-(2,2,2-trifluoroethoxy)-2-pyridyl]methyl]sulfinyl]-1H-benzimidazole in living organisms and shows anti-ulcer activity and the like, a production method thereof and use thereof.

### Disclosure of the Invention

The present inventors have first synthesized a compound represented by the following formula (I) and first found that this compound has unexpectedly superior stability to acid, gradually eliminates the substituent on the nitrogen atom of benzimidazole ring and affords a sustained acid secretion-suppressive action. Further studies based on these findings have resulted in the completion of the present invention.

According to the present invention, 2-[[[3-methyl-4-(2,2,2-trifluoroethoxy)-2-pyridyl]methyl]sulfinyl]-1H-benzimidazole is modified to give a prodrug (the compound of the formula (I)) stable to acid, which enables oral administration of the compound as a conventional tablet and the like without formulating an enteric-coated preparation. This has a consequence that the cost for formulating an enteric-coated preparation can be eliminated and the preparation of tablet and the like can be made smaller. A smaller preparation is advantageous in that it is easily swallowed by patients having difficulty in swallowing, particularly the elderly and children. In addition, absorption is rapid due to the absence of a sustained release effect afforded by enteric-coated preparations, expression of a gastric acid secretion-suppressive action is rapid, and alleviation of symptoms such as pain and the like is rapid. Furthermore, because the compound is gradually converted to a proton pump inhibitor in living organisms, a sustainable anti-ulcer agent and the like can be provided.

Accordingly, the present invention provides the following.
1) A benzimidazole compound represented by the formula (I) 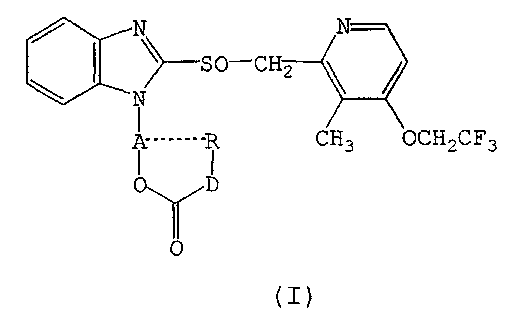 wherein A is an alkylidene group having 2 or more carbon atoms and optionally having substituent(s), R is a hydrocarbon group optionally having substituent(s) or a heterocyclic group optionally having substituent(s), or A and R may be bonded to each other to form a 4- to 8-membered ring optionally having substituent(s), and D is an oxygen atom or a bond
   (hereinafter sometimes abbreviated as compound (I)), or a salt thereof.
2) The compound of the above-mentioned 1) wherein (i) A and R are bonded to each other to form a 4- to 8-membered ring optionally having substituent(s) and D is an oxygen atom or a bond, or
   (ii) A and R are not bonded to each other, A is an alkylidene group having 2 or more carbon atoms and optionally having substituent(s), R is a hydrocarbon group optionally having substituent(s) or a heterocyclic group optionally having substituent(s) and D is an oxygen atom or a bond.
3) The compound of the above-mentioned 1), which is an (R)-form represented by the formula 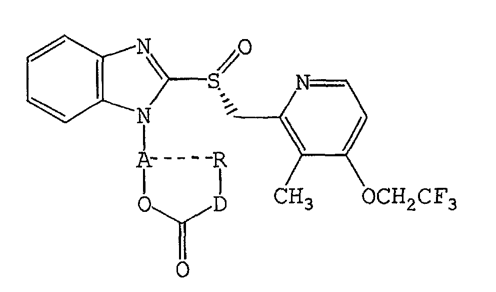 wherein each symbol is as defined in the above-mentioned 1).
4) The compound of the above-mentioned 1), wherein R is a C₁₋₆ alkyl group, a C₂₋₆ alkenyl group or a C₂₋₆ alkynyl group, which optionally has substituent(s) selected from the group consisting of (i) a C₆₋₁₄ aryl group, (ii) a hydroxy group, (iii) a halogen, (iv) a C₁₋₆ alkoxy group optionally substituted by halogen, (v) a C₇₋₁₂ aralkyloxy group, (vi) a C₁₋₅ alkoxy-carbonyl group and (vii) an acylamino group, or a C₃₋₈ cycloalkyl group or a C₆₋₁₄ aryl group, which optionally has substituent(s) selected from the group consisting of (i) a C₆₋₁₄ aryl group, (ii) a hydroxy group, (iii) a halogen, (iv) a C₁₋₆ alkoxy group optionally substituted by halogen, (v) a C₇₋₁₂ aralkyloxy group, (vi) a C₁₋₅ alkoxy-carbonyl group and (vii) a C₁₋₆ alkyl group optionally substituted by halogen, and A is a C₂₋₆ alkylidene group optionally substituted by halogen.
5) The compound of the above-mentioned 1), wherein R is a C₁₋₆ alkyl group optionally having substituent(s) selected from the group consisting of (i) a C₆₋₁₄ aryl group, (ii) a hydroxy group, (iii) a halogen, (iv) a C₁₋₆ alkoxy group optionally substituted by halogen, (v) a C₇₋₁₂ aralkyloxy group, (vi) a C₁₋₅ alkoxy-carbonyl group and (vii) an acylamino group or a C₃₋₈ cycloalkyl group or a C₆₋₁₄ aryl group, which optionally has substituent(s) selected from the group consisting of (i) a C₆₋₁₄ aryl group, (ii) a hydroxy group, (iii) a halogen, (iv) a C₁₋₆ alkoxy group optionally substituted by halogen, (v) a C₇₋₁₂ aralkyloxy group, (vi) a C₁₋₅ alkoxy-carbonyl group and (vii) a C₁₋₆ alkyl group optionally substituted by halogen, and A is a C₂₋₆ alkylidene group optionally substituted by halogen.
6) The compound of the above-mentioned 1), wherein the 4- to 8-membered ring formed by A and R bonded to each other is a ring represented by the formula 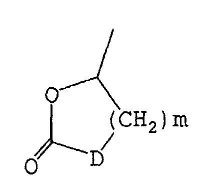 wherein m is an integer of 1 to 3, and other symbols are as mentioned above.
7) A benzimidazole compound represented by the formula (I') 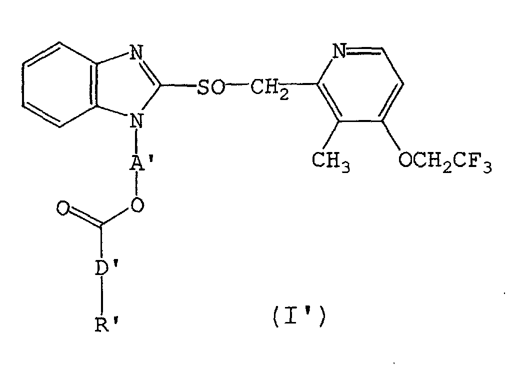 wherein A' is an alkylidene group having 2 or more carbon atoms and optionally having substituent(s), R' is a hydrocarbon group optionally having substituent(s) and D' is an oxygen atom or a bond, or a salt thereof.
8) The compound of the above-mentioned 7), which is an (R)-form represented by the formula 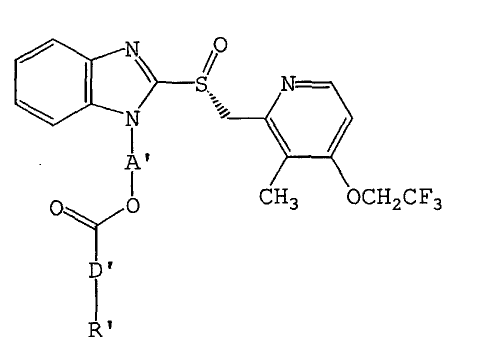 wherein each symbol is as defined in the above-mentioned 7).
9) The compound of the above-mentioned 7), wherein R' is a C₁₋₆ alkyl group, a C₂₋₆ alkenyl group or a C₂₋₆ alkynyl group, which optionally has substituent(s) selected from the group consisting of (i) a C₆₋₁₄ aryl group, (ii) a hydroxy group, (iii) a halogen, (iv) a C₁₋₆ alkoxy group optionally substituted by halogen, (v) a C₇₋₁₂ aralkyloxy group and (vi) a C₁₋₅ alkoxy-carbonyl group, or
   a C₃₋₈ cycloalkyl group or a C₆₋₁₄ aryl group, which optionally has substituent(s) selected from the group consisting of (i) a C₆₋₁₄ aryl group, (ii) a hydroxy group, (iii) a halogen, (iv) a C₁₋₆ alkoxy group optionally substituted by halogen, (v) a C₇₋₁₂ aralkyloxy group, (vi) a C₁₋₅ alkoxy-carbonyl group and (vii) a C₁₋₆ alkyl group optionally substituted by halogen, and A' is a C₂₋₆ alkylidene group optionally substituted by halogen.
10) The compound of the above-mentioned 7), wherein R' is a C₁₋₆ alkyl group optionally having substituent(s) selected from the group consisting of (i) a C₆₋₁₄ aryl group, (ii) a hydroxy group, (iii) a halogen, (iv) a C₁₋₆ alkoxy group optionally substituted by halogen, (v) a C₇₋₁₂ aralkyloxy group and (vi) a C₁₋₅ alkoxy-carbonyl group, or
   a C₃₋₈ cycloalkyl group or a C₆₋₁₄ aryl group, which optionally has substituent(s) selected from the group consisting of (i) a C₆₋₁₄ aryl group, (ii) a hydroxy group, (iii) a halogen, (iv) a C₁₋₆ alkoxy group optionally substituted by halogen, (v) a C₇₋₁₂ aralkyloxy group, (vi) a C₁₋₅ alkoxy-carbonyl group and (vii) a C₁₋₆ alkyl group optionally substituted by halogen, and A' is a C₂₋₆ alkylidene group optionally substituted by halogen.
11) The compound of the above-mentioned 7), wherein A' is an ethylidene group or a propylidene group.
12) The compound of the above-mentioned 7), wherein A' is an ethylidene group.
13) The compound of the above-mentioned 7), wherein A' is an ethylidene group and R' is a C₁₋₆ alkyl group or a C₃₋₈ cycloalkyl group.
14) A production method of the compound of the above-mentioned 1) or a salt thereof, which comprises (1) condensing a compound represented by the formula (II) 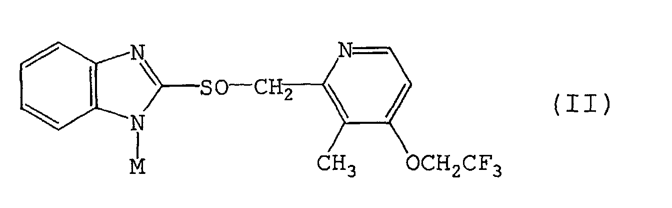 wherein M is a hydrogen atom, a metal cation or a quaternary ammonium ion (hereinafter sometimes abbreviated as compound (II)), or a salt thereof, with a compound represented by the formula (III) 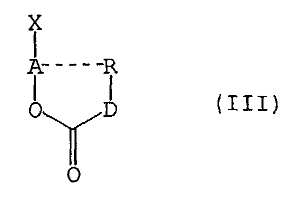 wherein X is a leaving group, A is an alkylidene group having 2 or more carbon atoms and optionally having substituent(s), R is a hydrocarbon group optionally having substituent(s) or a heterocyclic group optionally having substituent(s), or A and R may be bonded to each other to form a 4- to 8-membered ring optionally having substituent(s), and D is an oxygen atom or a bond (hereinafter sometimes abbreviated as compound (III)), or (2) subjecting a compound represented by the formula (IV) 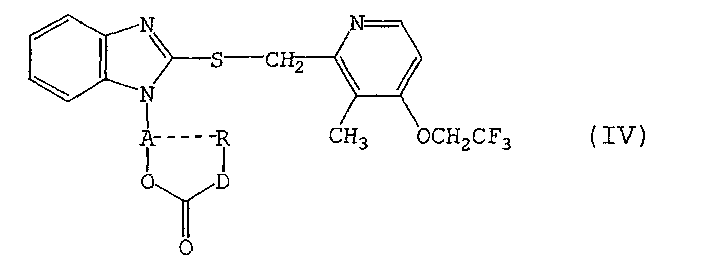 wherein each symbol is as defined above (hereinafter sometimes abbreviated as compound (IV)), or a salt thereof, to an oxidation reaction.
15) A pharmaceutical composition comprising the compound of the above-mentioned 1) or 7).
16) The pharmaceutical composition of the above-mentioned 15), which is an agent for the prophylaxis or treatment of peptic ulcer, gastritis, reflux esophagitis, symptomatic gastroesophageal reflux disease (symptomatic GERD), NUD, gastric cancer, gastric MALT lymphoma, Zollinger-Ellison syndrome, gastric hyperacidity or upper gastrointestinal hemorrhage.
17) The pharmaceutical composition of the above-mentioned 15), which is an agent for the eradication of Helicobacter pylori.
18) A commercial package comprising the pharmaceutical composition of the above-mentioned 16) and a written matter associated therewith, the written matter stating that the pharmaceutical composition can or should be used for the prophylaxis or treatment of peptic ulcer, gastritis, reflux esophagitis, symptomatic gastroesophageal reflux disease (symptomatic GERD), NUD, gastric cancer, gastric MALT lymphoma, Zollinger-Ellison syndrome, gastric hyperacidity or upper gastrointestinal hemorrhage.
19) A commercial package comprising the pharmaceutical composition of the above-mentioned 17) and a written matter associated therewith, the written matter stating that the pharmaceutical composition can or should be used for the eradication of *Helicobacter pylori.*
20) A method for the prophylaxis or treatment of peptic ulcer, gastritis, reflux esophagitis, symptomatic gastroesophageal reflux disease (symptomatic GERD), NUD, gastric cancer, gastric MALT lymphoma, Zollinger-Ellison syndrome, gastric hyperacidity or upper gastrointestinal hemorrhage, which comprises administering the compound of the above-mentioned 1) or 7).
21) A method for eradicating *Helicobacter pylori,* which comprises administering the compound of the above-mentioned 1) or 7).
22) Use of the compound of the above-mentioned 1) or 7) for the production of an agent for the prophylaxis or treatment of peptic ulcer, gastritis, reflux esophagitis, symptomatic gastroesophageal reflux disease (symptomatic GERD), NUD, gastric cancer, gastric MALT lymphoma, Zollinger-Ellison syndrome, gastric hyperacidity or upper gastrointestinal hemorrhage.
23) Use of the compound of the above-mentioned 1) or 7) for the production of an agent for the eradication of *Helicobacter pylori.*

### Embodiment of the Invention

In the present invention, the "C₆₋₁₄ aryl group" is a monocyclic or condensed polycyclic aromatic hydrocarbon group having 6 to 14 carbon atoms. Examples thereof include phenyl, naphthyl, anthryl, phenanthryl and acenaphthylenyl. Preferred is an aromatic hydrocarbon group having 6 to 10 carbon atoms, and for R, phenyl is particularly preferable.

In the present invention, the "halogen" is fluorine, chlorine, bromine or iodine. The halogen as a substituent of the hydrocarbon group represented by R and alkylidene group for A in the formula (I) is preferably fluorine or chlorine.

In the present invention, the "C₁₋₆ alkoxy group optionally substituted by halogen" is a linear or branched chain alkoxy group having 1 to 6 carbon atoms, which is optionally substituted by halogen (as defined above, preferably 1 to 5, more preferably 1 to 3, halogens). Examples of the C₁₋₆ alkoxy group include methoxy, ethoxy, n-propoxy, isopropoxy, n-butoxy, isobutoxy, sec-butoxy, tert-butoxy and the like, with preference given to an alkoxy group having 1 to 4 carbon atoms. As the C₁₋₆ alkoxy group optionally substituted by halogen, methoxy, ethoxy, n-propoxy, isopropoxy, trifluoromethoxy and 2,2,2-trifluoroethoxy are preferable.

In the present invention, the "C₁₋₆ alkyl group" means a linear or branched chain alkyl group having 1 to 6 carbon atoms. Examples thereof include methyl, ethyl, n-propyl, isopropyl, n-butyl, isobutyl, sec-butyl, tert-butyl, n-pentyl, isopentyl, neopentyl, 1-methylpropyl, n-hexyl, isohexyl, 1,1-dimethylbutyl, 2,2-dimethylbutyl, 3,3-dimethylbutyl, 3,3-dimethylpropyl, 2-ethylbutyl and the like, with preference given to alkyl group having 1 to 4 carbon atoms. For R, ethyl, isopropyl and tert-butyl are preferable, and isopropyl is particularly preferable.

In the present invention, the "C₇₋₁₂ aralkyloxy group" is an aralkyloxy group having 7 to 12 carbon atoms, wherein the aryl group is as defined for the above-mentioned aryl group (preferably phenyl group) and the alkyl moiety is as defined for the above-mentioned "C₁₋₆ alkyl group". Examples thereof include benzyloxy, 1-naphthylmethyloxy, 2-naphthylmethyloxy and the like. Preferred is an aralkyloxy group having 7 to 11 carbon atoms, and particularly preferred is benzyloxy.

In the present invention, the "C₁₋₅ alkoxy-carbonyl group" is an alkoxycarbonyl group wherein the alkoxy moiety is a linear or branched chain alkoxy group having 1 to 5 carbon atoms, which is exemplified by methoxycarbonyl, ethoxycarbonyl, propoxycarbonyl, isopropoxycarbonyl and the like. Preferred is an alkoxycarbonyl group wherein the alkoxy moiety has 1 to 4 carbon atoms, and particularly preferred are methoxycarbonyl and ethoxycarbonyl.

In the present invention, the "C₁₋₆ alkyl group optionally substituted by halogen" is a C₁₋₆ alkyl group (as defined above) optionally substituted by halogen (as defined above, preferably 1 to 5, more preferably 1 to 3, halogens), which is preferably methyl, ethyl, propyl, isopropyl or trifluoromethyl.

The "alkylidene group having 2 or more carbon atoms" in the present invention is a linear or branched chain alkylidene group having 2 or more carbon atoms, which is, for example, ethylidene, propylidene, butylidene, pentylidene or hexylidene, preferably an alkylidene group having 2 to 6 carbon atoms, more preferably a linear alkylidene group having 2 or 3 carbon atoms. Of these, ethylidene and propylidene are preferable, and ethylidene is particularly preferable.

In the present invention, the "C₂₋₆ alkenyl group" is a linear or branched chain alkenyl group having 2 to 6 carbon atoms, which is exemplified by vinyl, n-propenyl, isopropenyl, n-butenyl, isobutenyl, sec-butenyl, tert-butenyl, n-pentenyl, isopentenyl, neopentenyl, 1-methylpropenyl, n-hexenyl, isohexenyl, 1,1-dimethylbutenyl, 2,2-dimethylbutenyl, 3,3-dimethylbutenyl, 3,3-dimethylpropenyl, 2-ethylbutenyl and the like. Preferred is an alkenyl group having 2 to 4 carbon atoms, and particularly preferred are vinyl, n-propenyl and isopropenyl.

In the present invention, the "C₂₋₆ alkynyl group" is a linear or branched chain alkynyl group having 2 to 6 carbon atoms, which is exemplified by ethynyl, n-propynyl (1-propynyl), isopropynyl (2-propynyl), n-butynyl, isobutynyl, sec-butynyl, tert-butynyl, n-pentynyl, isopentynyl, neopentynyl, 1-methylpropynyl, n-hexynyl, isohexynyl, 1,1-dimethylbutynyl, 2,2-dimethylbutynyl, 3,3-dimethylbutynyl, 3,3-dimethylpropynyl, 2-ethylbutynyl and the like. Preferred is an alkynyl group having 2 or 3 carbon atoms, and particularly preferred are ethynyl, 1-propynyl and 2-propynyl.

In the present invention, the "C₃₋₈ cycloalkyl group" is a linear or branched chain cycloalkyl group having 3 to 8 carbon atoms, which is exemplified by cyclopropyl, cyclobutyl, cyclopentyl, cyclohexyl, cycloheptyl, cyclooctyl and the like. Preferred is a cycloalkyl group having 5 to 7 carbon atoms, more preferred are cyclopentyl, cyclohexyl and cycloheptyl, and particularly preferred is cyclohexyl.

The "amino group optionally substituted by C₁₋₆ alkyl group" in the present invention means an amino group optionally substituted by the "C₁₋₆ alkyl group" as defined above. Examples thereof include methylamino, ethylamino, propylamino, isopropylamino, butylamino, sec-butylamino, tert-butylamino, pentylamino, hexylamino and the like, with preference given to methylamino, ethylamino, propylamino and isopropylamino.

The acyl group of the "acylamino group" in the present invention is preferably an alkanoyl group having 1 to 6, more preferably 1 to 3, carbon atoms. As such acylamino group, for example, formylamino, acetylamino, propionylamino and the like can be mentioned, with preference given to acetylamino.

In the present invention, the "hydrocarbon group" encompasses an aliphatic or aromatic hydrocarbon group, wherein the aliphatic hydrocarbon group means a saturated or unsaturated linear, branched chain or cyclic hydrocarbon group. As the hydrocarbon group, a hydrocarbon group having 1 to 14 carbon atoms is preferable, which is exemplified by a C₁₋₆ alkyl group, a C₂₋₆ alkenyl group, a C₂₋₆ alkynyl group, a C₃₋₈ cycloalkyl group and a C₆₋₁₄ aryl group. Preferred are a C₁₋₆ alkyl group, a C₃₋₈ cycloalkyl group and a C₆₋₁₄ aryl group and more preferred are a C₁₋₆ alkyl group and a C₃₋₈ cycloalkyl group.

In the present invention, the "metal cation" is exemplified by alkali metal ions (e.g., Na⁺, K⁺, Li⁺, Cs⁺ etc.), with preference given to Na⁺ and Cs⁺.

In the present invention, the "quaternary ammonium ion" is exemplified by tetramethylammonium, tetraethylammonium, tetrapropylammonium, tetrabutylammonium and the like, with particular preference given to tetrabutylammonium.

In the present invention, the "heterocyclic group" is a monovalent group, which is obtained by eliminating one optional hydrogen atom from a heterocycle having 1 to 3, preferably 1 or 2, hetero atoms (e.g., oxygen atom, nitrogen atom, sulfur atom, etc.) and may be saturated or unsaturated, and aromatic or aliphatic. Specific examples include tetrahydro-2H-pyranyl, 1,3-dioxanyl, 4-piperidinyl, tetrahydro-2H-thiopyranyl, tetrahydrofuranyl, pyrrolidinyl, pyridyl, pyrrolyl, furyl, thienyl and the like, with preference given to tetrahydro-2H-pyranyl and 1,3-dioxanyl. A and R in the formula (I) of the present invention may be bonded to each other to form a ring. As used herein, the ring is a 4- to 8-membered ring, preferably a 5- to 7-membered ring, which is a heterocycle containing at least one oxygen atom as a hetero atom (e.g., oxygen atom, nitrogen atom, sulfur atom etc.), and may be saturated or unsaturated. As such ring, for example, a ring represented by the formula 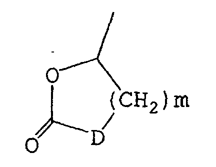 wherein the symbols in the formula are as defined above, can be mentioned. The 4- to 8-membered ring formed by A and R bonded to each other is bonded to the N-position of the imidazole ring of the above-mentioned lansoprazole, and applied as a prodrug of a proton pomp inhibitor (PPI), or bonded to a different pyridylmethylsulfonyl substituted benzimidazole structure such as omeprazole, pantoprazole, rabeprazole and the like, and preferably applied as a prodrug of PPI.

Specific examples of the 4- to 8-membered ring formed by A and R of the formula (I) bonded to each other in the present invention include 1,3-dioxolan-2-one, dihydrofuran-2(3H)-one, 1,3-dioxan-2-one, tetrahydro-2H-pyran-2-one and the like, with preference given to 1,3-dioxolan-2-one.

The "hydrocarbon group" of the present invention may be substituted, wherein the substituent is exemplified by a C₆₋₁₄ aryl group, a hydroxy group, a halogen, a C₁₋₆ alkoxy group optionally substituted by halogen, a C₇₋₁₂ aralkyloxy group, a C₁₋₅ alkoxy-carbonyl group, a C₁₋₆ alkyl group optionally substituted by halogen, an acylamino group, an amino group optionally substituted by C₁₋₆ alkyl group and the like. The number of the substituents is 1 to 5, preferably 1 to 3.

The "C₁₋₆ alkyl group", "C₂₋₆ alkenyl group" and "C₂₋₆ alkynyl group" in the hydrocarbon group of the present invention may be substituted, and the preferable substituent is exemplified by (i) a C₆₋₁₄ aryl group, (ii) a hydroxy group, (iii) a halogen, (iv) a C₁₋₆ alkoxy group optionally substituted by halogen, (v) a C₇₋₁₂ aralkyloxy group, (vi) a C₁₋₅ alkoxy-carbonyl group, (vii) an acylamino group, (viii) an amino group optionally substituted by C₁₋₆ alkyl group and the like. Of these, (i) to (vii) are preferable, and (i) to (vi) are more preferable. The number of the substituents is 1 to 5, preferably 1 to 3.

The above-mentioned "C₃₋₈ cycloalkyl group" and "C₆₋₁₄ aryl group" may be substituted, wherein the substituent is exemplified by (i) a C₆₋₁₄ aryl group, (ii) a hydroxy group, (iii) a halogen, (iv) a C₁₋₆ alkoxy group optionally substituted by halogen, (v) a C₇₋₁₂ aralkyloxy group, (vi) a C₁₋₅ alkoxy-carbonyl group, (vii) a C₁₋₆ alkyl group optionally substituted by halogen, (viii) an amino group optionally substituted by C₁₋₆ alkyl group and the like. Of these, (i) to (vii) are preferable. The number of the substituent(s) is 1 to 5, preferably 1 to 3.

The "alkylidene group having 2 or more carbon atoms" of the present invention may be substituted, wherein the substituent is exemplified by a halogen, a C₁₋₆ alkoxy group (same as the "C₁₋₆ alkoxy group" of the above-mentioned "C₁₋₆ alkoxy group optionally substituted by halogen") and the like, with particular preference given to a halogen.

R in the formula (I) is preferably a C₁₋₆ alkyl group, a C₂₋₆ alkenyl group or a C₂₋₆ alkynyl group, which optionally has substituent(s) selected from the group consisting of (i) a C₆₋₁₄ aryl group, (ii) a hydroxy group, (iii) a halogen, (iv) a C₁₋₆ alkoxy group optionally substituted by halogen, (v) a C₇₋₁₂ aralkyloxy group, (vi) a C₁₋₅ alkoxy-carbonyl group and (vii) an acylamino group, or
a C₃₋₈ cycloalkyl group or a C₆₋₁₄ aryl group, which optionally has substituent(s) selected from the group consisting of (i) a C₆₋₁₄ aryl group, (ii) a hydroxy group, (iii) a halogen, (iv) a C₁₋₆ alkoxy group optionally substituted by halogen, (v) a C₇₋₁₂ aralkyloxy group, (vi) a C₁₋₅ alkoxy-carbonyl group and (vii) a C₁₋₆ alkyl group optionally substituted by halogen, more preferably (1) a C₁₋₆ alkyl group, (2) a C₆₋₁₄ aryl group or (3) a C₃₋₈ cycloalkyl group, which optionally has the above-mentioned substituent(s).

R in the formula (I) and R' in the formula (I') are more preferably a C₁₋₆ alkyl group, a C₂₋₆ alkenyl group or a C₂₋₆ alkynyl group, which optionally has substituent(s) selected from the group consisting of (i) a C₆₋₁₄ aryl group, (ii) a hydroxy group, (iii) a halogen, (iv) a C₁₋₆ alkoxy group optionally substituted by halogen, (v) a C₇₋₁₂ aralkyloxy group and (vi) a C₁₋₅ alkoxy-carbonyl group, or
a C₃₋₈ cycloalkyl group or a C₆₋₁₄ aryl group, which optionally has substituent(s) selected from the group consisting of (i) a C₆₋₁₄ aryl group, (ii) a hydroxy group, (iii) a halogen, (iv) a C₁₋₆ alkoxy group optionally substituted by halogen, (v) a C₇₋₁₂ aralkyloxy group, (vi) a C₁₋₅ alkoxy-carbonyl group and (vii) a C₁₋₆ alkyl group optionally substituted by halogen, more preferably (1) a C₁₋₆ alkyl group, (2) a C₆₋₁₄ aryl group or (3) a C₃₋₈ cycloalkyl group, which optionally has the above-mentioned substituent(s).

R in the formula (I) may be the hetero ring as mentioned above, and the hetero ring may have substituent(s). As the substituent, those mentioned above as the substituents of the "hydrocarbon group", a C₁₋₆ alkyl group, a C₂₋₆ alkenyl group, a C₂₋₆ alkynyl group and the like can be mentioned. Of these substituents, 1 to 5, preferably 1 to 3, may be used.

As A, a C₂₋₆ alkylidene group optionally substituted by halogen is preferable, an ethylidene group and a propylidene group are more preferable, and an ethylidene group is particularly preferable.

As preferable embodiments of compound (I) of the present invention, the following compounds can be mentioned.
(a) A compound wherein A and R are not bonded to each other, A is an alkylidene group having 2 or more carbon atoms and optionally having substituent(s), R is a hydrocarbon group optionally having substituent(s), and D is an oxygen atom or a bond (same as the above-mentioned compound of the formula (I')) is preferable, and particularly, a compound wherein A is an ethylidene group, D is an oxygen atom or a bond, and R is a C₁₋₆ alkyl group or a C₃₋₈ cycloalkyl group is more preferable.
(b) A compound wherein A and R are bonded to each other to form a 4- to 8-membered ring represented by the formula 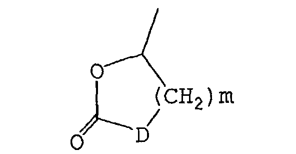
wherein each symbol as defined above, is preferable, and a compound wherein D is an oxygen atom is particularly preferable. Specifically, a compound wherein m is 1 is preferable.

In compound (I), a pharmacologically acceptable basic salt can be formed between an acidic group in a molecule and an inorganic base, an organic base and the like. As used herein, the basic salt is, for example, an inorganic basic salt (e.g., salt with alkali metal (e.g., sodium, potassium etc.), alkaline earth metal (e.g., calcium etc.), ammonia etc.), an organic basic salt (e.g., salt with dimethylamine, triethylamine, piperazine, pyrrolidine, piperidine, 2-phenylethylamine, benzylamine, ethanolamine, diethanolamine, pyridine, collidine etc.) and the like.

For compound (I), a pharmacologically acceptable acid addition salt can be formed between a basic group in a molecule and an inorganic acid, an organic acid and the like. As used herein, the acid addition salt is, for example, an inorganic acid salt (e.g., hydrochloride, sulfate, hydrobromide, phosphate etc.), an organic acid salt (e.g., acetate, trifluoroacetate, succinate, maleate, fumarate, propionate, citrate, tartrate, lactate, oxalate, methanesulfonate, p-toluenesulfonate etc.) and the like.

The compound (I) of the present invention encompasses hydrates. Examples of the "hydrate" include 0.5 hydrate - 5.0 hydrate. Of these, 0.5 hydrate, 1.0 hydrate, 1.5 hydrate and 2.0 hydrate are preferable.

The compound (I) of the present invention encompasses racemates, optically active compounds and a mixture thereof. As the optically active compound, such compound wherein one enantiomer is in enantiomer excess (e.e.) of not less than 90% is preferable, more preferably in enantiomer excess (e.e.) of not less than 99%. As an optically active form, an (R)-form represented by the formula 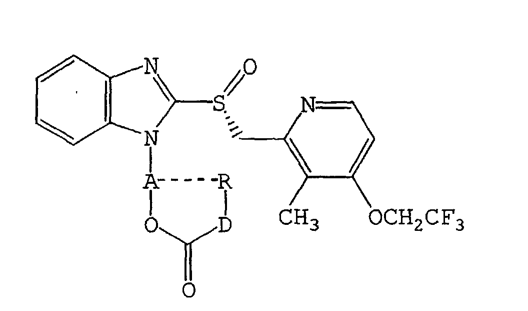 wherein each symbol is as defined above, is preferable.

In the definition of A and R in the formula (I) of the present invention, the "A is an alkylidene group having 2 or more carbon atoms and optionally having substituent(s), R is a hydrocarbon group optionally having substituent(s) or a heterocyclic group optionally having substituent(s), or A and R may be bonded to each other to form a 4- to 8-membered ring optionally having substituent(s)" includes (i) an embodiment wherein A and R are bonded to each other to form a 4- to 8-membered ring optionally having substituent(s) and D is an oxygen atom or a bond, and (ii) an embodiment wherein A and R are not bonded to each other, A is an alkylidene group having 2 or more carbon atoms and optionally having substituent(s), and R is a hydrocarbon group optionally having substituent(s) or a heterocyclic group optionally having substituent(s).

The compound (I) can be produced by a method known per se, such as the methods described in US Patent Nos. 4,873,337 and 5,021,433, or a similar method, such as the following methods A and B.

### Method A

The compound (II) or a salt thereof is condensed with compound (III), whereby compound (I) or a salt thereof can be obtained. 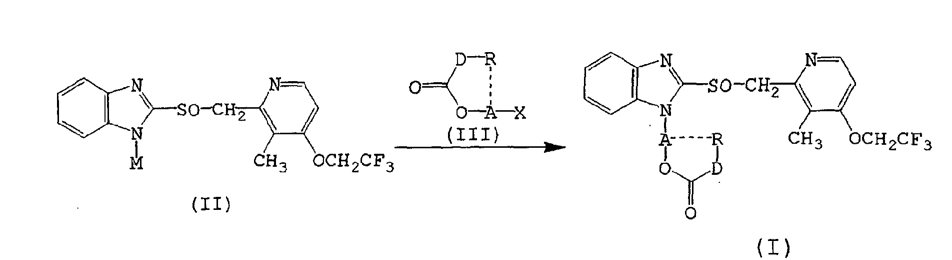 wherein M is a hydrogen atom, a metal cation or a quaternary ammonium ion, X is a leaving group, and other symbols are as defined above.

As the leaving group for X, a halogen atom (chlorine atom, bromine atom, fluorine atom, iodine atom), a C₁₋₆ alkylsulfonyloxy group (e.g., methanesulfonyloxy, ethanesulfonyloxy etc.) and a C₆₋₁₀ arylsulfonyloxy group (e.g., benzenesulfonyloxy, p-toluenesulfonyloxy etc.) can be mentioned.

For example, Method A is performed by reacting compound (II) or a salt thereof with compound (III) in the presence of a base. To be specific, for example, a base is added to a mixed solution of compound (II) or a salt thereof and compound (III), and the mixture is stirred.

The salt of compound (II) here is exemplified by those similar to the above-mentioned salts of compound (I), which are acid addition salts such as inorganic acid salts (e.g., hydrochloride, sulfate, hydrobromide, phosphate etc.), organic acid salts (e.g., acetate, trifluoroacetate, succinate, maleate, fumarate, propionate, citrate, tartrate, lactate, oxalate, methanesulfonate, p-toluenesulfonate etc.), and the like.

The reaction of Method A is generally conducted in a solvent, and a solvent that does not inhibit the above-mentioned reaction is selected as appropriate. Examples of such solvent include alcohols (e.g., methanol, ethanol, propanol, isopropanol, butanol, tert-butanol etc.), ethers (e.g., dioxane, tetrahydrofuran, diethyl ether, tert-butyl methyl ether, diisopropyl ether, ethylene glycol dimethyl ether etc.), esters (e.g., ethyl formate, ethyl acetate, n-butyl acetate etc.), halogenated hydrocarbons (e.g., dichloromethane, chloroform, carbon tetrachloride, trichlene, 1,2-dichloroethane etc.), hydrocarbons (e.g., n-hexane, benzene, toluene etc.), amides (e.g., formamide, N,N-dimethylformamide, N,N-dimethylacetamide etc.), ketones (e.g., acetone, methyl ethyl ketone, methyl isobutyl ketone etc.), nitriles (e.g., acetonitrile, propionitrile etc.) and the like, as well as dimethyl sulfoxide, sulfolane, hexamethylphosphoramide, water and the like, which may be used alone or as a mixed solvent. The amount of the solvent to be used is not particularly limited as long as the reaction mixture can be stirred, which is generally a 2- to 100-fold amount by weight of compound (II) or a salt thereof.

The base in Method A is, for example, an inorganic base such as C₁₋₆ alkyl lithium or C₆₋₁₀ aryl lithium (e.g., methyl lithium, ethyl lithium, n-butyl lithium, sec-butyl lithium, tert-butyl lithium, phenyl lithium etc.), lithium C₂₋₆ alkylamides (e.g., lithium dimethylamide, lithium diethylamide, lithium diisopropylamide etc.), metal hydrides (e.g., lithium hydride, sodium hydride etc.), alkali metal C₁₋₆ alkoxides (e.g., lithium ethoxide, lithium tert-butoxide, sodium methoxide, sodium ethoxide, potassium tert-butoxide etc.), alkali metal amides (e.g., lithium amide, potassium amide, sodium amide etc.), alkali metal hydroxides (e.g., lithium hydroxide, potassium hydroxide, sodium hydroxide etc.), carbonates or bicarbonates of alkali metal (e.g., sodium carbonate, potassium carbonate, cesium carbonate, sodium hydrogen carbonate etc.) and the like; organic bases such as tertiary amine (e.g., triethylamine, tri(n-propyl) amine, tri(n-butyl)amine, diisopropylethylamine, cyclohexyldimethylamine, pyridine, 4-(dimethylamino)pyridine, lutidine, γ-collidine, N,N-dimethylaniline, N-methylpiperidine, N-methylpyrrolidine, N-methylmorpholine etc.) and the like. The lower limit of the amount of the base to be used is generally not less than 0.1 mol, preferably not less than 0.5 mol, more preferably not less than 1 mol, and the upper limit is generally not more than 10 mol, preferably not more than 3 mol, relative to 1 mol of compound (II) or a salt thereof.

In Method A, generally 1 mol - 5 mol, preferably 1 mol - 3 mol, of compound (III) can be used per 1 mol of compound (II).

Method A is generally performed at a temperature of from about -80°C to 100°C, preferably 0°C to 60°C, depending on the kinds of compounds (II) and (III) and the solvent, reaction temperature and the like, and generally completes in 1 min.-72 hrs., preferably 15 min. - 24 hrs.

The compound (II) can be produced according to the method described in JP-A-61-50978, USP 4,628,098 and the like or a method similar thereto.

The compound (III) is produced according to the methods described in Journal of The American Chemical Society, vol. 43, p. 651 (1921), JP-B-61-40246, JP-B-4-58460 and Journal of The American Chemical Society, vol. 105, p. 7592 (1983) or a method analogous thereto.

When X of compound (III) is other than iodine, lithium iodide, sodium iodide and the like are used to convert compound (III) to compound (III) wherein X is iodine, which can be then subjected to a reaction with compound (II).

### Method B

The compound (I) or a salt thereof can be obtained by subjecting compound (IV) or a salt thereof to an oxidation reaction. 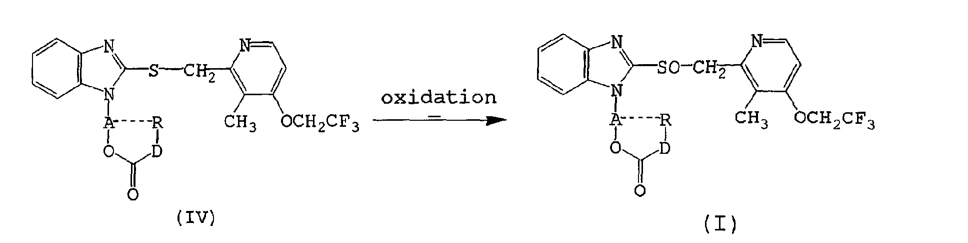 wherein each symbol is as defined above.

For example, the reaction in Method B can be carried out using an oxidant such as nitric acid, hydrogen peroxide, peroxy acid (e.g., magnesium monoperoxyphthalic acid etc.), peroxy acid ester, ozone, dinitrogen tetraoxide, iodosobenzene, N-halosuccinimide, 1-chlorobenzotriazole, tert-butyl hypochlorite, diazabicyclo[2.2.2]octane-bromine complex, sodium metaperiodate, selenium dioxide, manganese dioxide, chromic acid, cerium ammonium nitrate, bromine, chlorine, sulfuryl chloride, 3-chloroperoxybenzoic acid and the like. The amount of the oxidant to be used is generally 0.5 mol - 2 mol, preferably 0.8 mol - 1.2 mol, per 1 mol of compound (IV) or a salt thereof. When the oxidation is carried out using the above-mentioned hydrogen peroxide or peroxy acid as an oxidant, it can be also performed in the presence of a catalyst such as vanadium acetate and the like.

The reaction of Method B is generally carried out in a solvent inert to the above-mentioned oxidation reaction. Examples of the "solvent inert to the above-mentioned oxidation reaction" include water, alcohols (e.g., methanol, ethanol, 1-propanol, 2-propanol etc.), ketones (e.g., acetone, methyl ethyl ketone etc.), nitriles (e.g., acetonitrile, propionitrile etc.), amides (e.g., formamide, N,N-dimethylformamide etc.), ethers (e.g., diethyl ether, tert-butyl methyl ether, diisopropyl ether, dioxane, tetrahydrofuran etc.), sulfoxides (e.g., dimethyl sulfoxide etc.), polar solvents (e.g., sulfolane, hexamethylphosphoramide etc.), halogenated hydrocarbons (e.g., dichloromethane, etc.) and hydrocarbons (e.g., n-hexane, benzene, toluene, etc.), which may be used alone or as a mixed solvent thereof. The "solvent inert to the above-mentioned oxidation reaction" is used in an amount of generally not less than a 1-fold amount by weight of compound (IV) or a salt thereof for the lower limit, and generally not more than a 200-fold amount by weight of compound (IV) or a salt thereof, preferably not more than a 100-fold amount by weight of compound (IV) or a salt thereof for the upper limit.

The reaction of Method B is generally carried out at a temperature of from -80°C to 80°C, preferably 0°C to 30°C and generally completes in 1 min. - 6 hrs., preferably 15 min. - 1 hr.

For example, the compound (IV), which is a starting material of Method B, can be obtained by subjecting 2-[[[3-methyl-4-(2,2,2-trifluoroethoxy)-2-pyridyl]methyl]thio]-1H-benzimidazole to a reaction similar to the reaction in Method A. Namely, compound (II'), wherein the -S(=O)- group of compound (II) has been converted to an -S- group, is reacted with compound (III) under the conditions similar to those in the above-mentioned method A to give compound (IV). When compound (II') and compound (III) are reacted, the reaction may be carried out in the co-presence of a catalyst such as 18-crown-6. In addition, when X of compound (III) is other than iodine, it is possible to convert X of compound (III) to iodine using lithium iodide, sodium iodide and the like, and react the resulting compound (III) with compound (II').

The salt of compound (IV) is exemplified by the above-mentioned salts of the compound (I), which are acid addition salts such as inorganic acid salt (e.g., hydrochloride, sulfate, hydrobromide, phosphate etc.), organic acid salts (e.g., acetate, trifluoroacetate, succinate, maleate, fumarate, propionate, citrate, tartrate, lactate, oxalate, methanesulfonate, p-toluenesulfonate etc.) and the like.

The compound (I) and a salt thereof obtained by the above-mentioned methods A and B can be isolated and purified from the reaction mixture by a separation means known *per se* (e.g., concentration, concentration under reduced pressure, solvent extraction, crystallization, recrystallization, phase transfer, chromatography etc.).

One of the starting materials of Method A, (R)-2-[[[3-methyl-4-(2,2,2-trifluoroethoxy)-2-pyridyl]methyl]sulfinyl]-1H-benzimidazole, can be produced by, for example, (A) subjecting 2-[[[3-methyl-4-(2,2,2-trifluoroethoxy)-2-pyridyl]methyl]sulfinyl]-1H-benzimidazole or a salt thereof to optical resolution, (B) asymmetric oxidation of 2-[[[3-methyl-4-(2,2,2-trifluoroethoxy)-2-pyridyl]methyl]thio]-1H-benzimidazole or (C) the production method described in WO00/78745, WO01/83473 and the like, and the like.

The method of optical resolution of (A) includes methods known *per se,* such as fractional recrystallization methods, chiral column methods, diastereomer methods, and so forth.

Asymmetric oxidation of (B) includes methods known per se, such as the method described in WO96/02535 and the like.

The "fractional recrystallization method" includes a method in which a salt is formed between a racemate and an optically active compound [e.g., (+)-mandelic acid, (-)-mandelic acid, (+)-tartaric acid, (-)-tartaric acid, (+)-1-phenethylamine, (-)-1-phenethylamine, cinchonine, (-)-cinchonidine, brucine etc.], which salt is separated by fractional recrystallization etc. and, if desired, subjected to a neutralization process to give a free optical isomer.

The "chiral column method" includes a method in which a racemate or a salt thereof is applied to a column for optical isomer separation (chiral column). In the case of liquid chromatography, for example, optical isomers are separated by adding a racemate to a chiral column such as ENANTIO-OVM (produced by Tosoh Corporation), the CHIRAL series (produced by Daicel Corporation) and the like, and developing the racemate in water, a buffer (e.g., phosphate buffer), an organic solvent (e.g., hexane, ethanol, methanol, isopropanol, acetonitrile, trifluoroacetic acid, diethylamine, triethylamine etc.), or a solvent mixture thereof. In the case of gas chromatography, for example, a chiral column such as CP-Chirasil-DeX CB (produced by GL Science) and the like is used to separate optical isomers.

The "diastereomer method" includes a method in which a racemate and an optically active reagent are reacted (preferably, an optically active reagent is reacted with the group at the 1-position of the benzimidazole group) to give a diastereomeric mixture, which is then subjected to ordinary separation means (e.g., fractional recrystallization, chromatography etc.) to obtain either diastereomer, which is subjected to a chemical reaction (e.g., acid hydrolysis, base hydrolysis, hydrogenolysis etc.) to cut off the optically active reagent moiety, whereby the desired optically active compound is obtained. Said "optically active reagent" includes, for example, optically active organic acids such as MTPA [α-methoxy-α-(trifluoromethyl)phenylacetic acid], (-)-menthoxyacetic acid and the like, optically active alkoxymethyl halides such as (1R-endo)-2-(chloromethoxy)-1,3,3-trimethylbicyclo[2.2.1]heptane and the like, and the like. The compound (I) and a salt thereof of the present invention may be racemates or optically active compounds. They are particularly preferably in an (R)-form.

The compound (I) and a salt thereof of the present invention are converted to 2-[[[3-methyl-4-(2,2,2-trifluoroethoxy)-2-pyridyl]methyl]sulfinyl]-1H-benzimidazole (hereinafter sometimes to be referred to as lansoprazole), which is a conventionally known proton pump inhibitor, in living organisms, and are useful as pharmaceutical agents, because they have a superior anti-ulcer activity, a gastric acid secretion-suppressive action, a mucosa-protecting action, an anti-*Helicobacter pylori* action and the like, and show low toxicity. In addition, since they are stable to acid, they do not require formulation into an enteric-coated preparation for oral administration, which in turn eliminates the cost for formulating enteric-coated preparation. Moreover, the tablet can be made smaller, which is easily swallowed by patients having difficulty in swallowing, particularly the elderly and children. Furthermore, since absorption is faster than in enteric-coated preparations, expression of gastric acid secretion-suppressive action is rapid. The preparation is long-acting because it is gradually converted to a conventionally known proton pump inhibitor in living organisms. Consequently, the compounds are useful as anti-ulcer agents and the like.

The compound (I) and a salt thereof of the present invention are useful for the prophylaxis or treatment of peptic ulcer (e.g., gastric ulcer, gastric ulcer due to post-operative stress, duodenal ulcer, anastomotic ulcer, Zollinger-Ellison syndrome, ulcer caused by nonsteroidal antiinflammatory etc.); gastritis; reflux esophagitis; symptomatic gastroesophageal reflux disease (symptomatic GERD); NUD (Non Ulcer Dyspepsia); gastric cancer (including gastric cancer due to promoted production of interleukin-1β caused by genetic polymorphism of interleukin-1); gastric MALT lymphoma; Zollinger-Ellison syndrome; gastric hyperacidity (e.g. gastric hyperacidity due to post-operative stress); hemorrhage of upper gastrointestinal tract caused by acute stress ulcer, hemorrhagic gastritis, invasion stress (stress due to major surgery requiring intensive management after operation and cerebrovascular disorder, external injury in the head, multiple organ failure and extensive burn requiring intensive treatment) etc., and the like, pre-anesthetic administration, eradication of *Helicobacter pylori*, and the like, in mammals (e.g., human, simian, sheep, cattle, horse, dog, cat, rabbit, rat, mouse etc.).

The content of compound (I) or a salt thereof of the present invention in the pharmaceutical composition of the present invention is about 0.01 to 100% by weight relative to the entire composition. Though subject to change depending on the administration subject, administration route, target disease and the like, its dose is about 0.5 to 1,500 mg/day, preferably about 5 to 150 mg/day, based on the active ingredient, when, for example, the compound is orally administered as an anti-ulcer agent to an adult human (60 kg). The compound (I) or a salt thereof of the present invention may be administered once daily or in 2 or 3 divided portions per day.

The compound (I) and a salt thereof of the present invention show low toxicity and can be safely administered orally or parenterally (e.g., topical, rectal, intravenous administrations etc.) as they are or as a preparation containing a pharmaceutical composition containing a pharmacologically acceptable carrier admixed according to a method known per se, such as tablets (including sugar-coated tablets and film-coated tablets), powder, granule, capsule (including soft capsule), orally disintegrating tablet, liquid, injection, suppository, sustained-release preparation, plaster and the like. Particularly, they are preferably administered as oral agents in the form of tablet, granules, capsule and the like.

The pharmacologically acceptable carrier that may be used to produce the pharmaceutical composition of the present invention includes various organic or inorganic carrier substances in common use as pharmaceutical materials, including excipients, lubricants, binders, disintegrants, water-soluble polymers and basic inorganic salts for solid preparations; and solvents, dissolution aids, suspending agents, isotonizing agents, buffers and soothing agents for liquid preparations and the like. Other ordinary additives such as preservatives, anti-oxidants, coloring agents, sweetening agents, souring agents, bubbling agents, flavors etc. may be also used as necessary.

Such "excipients" include, for example, lactose, sucrose, D-mannitol, starch, cornstarch, crystalline cellulose, light silicic anhydride, titanium oxide and the like.

Such "lubricants" include, for example, magnesium stearate, sucrose fatty acid esters, polyethylene glycol, talc, stearic acid and the like.

Such "binders" include, for example, hydroxypropyl cellulose, hydroxypropylmethyl cellulose, crystalline cellulose, starch, polyvinylpyrrolidone, gum arabic powder, gelatin, pullulan, low-substituted hydroxypropyl cellulose and the like.

Such "disintegrants" include (1) crosslinked povidone, (2) what is called super-disintegrants such as crosslinked carmellose sodium (FMC-Asahi Chemical) and carmellose calcium (Gotoku Yakuhin) etc., (3) carboxymethyl starch sodium (e.g., product of Matsutani Chemical), (4) low-substituted hydroxypropyl cellulose (e.g., product of Shin-Etsu Chemical), (5) corn starch, and so forth. Said "crosslinked povidone" may be any crosslinked polymer having the chemical name 1-ethenyl-2-pyrrolidinone homopolymer, including polyvinylpolypyrrolidone (PVPP) and 1-vinyl-2-pyrrolidinone homopolymer, and is exemplified by Colidon CL (produced by BASF), Polyplasdon XL (produced by ISP), Polyplasdon XL-10 (produced by ISP), Polyplasdon INF-10 (produced by ISP) and the like.

Such "water-soluble polymers" include, for example, ethanol-soluble water-soluble polymers [e.g., cellulose derivatives such as hydroxypropyl cellulose (hereinafter also referred to as HPC) etc., polyvinylpyrrolidone and the like], ethanol-insoluble water-soluble polymers [e.g., cellulose derivatives such as hydroxypropylmethyl cellulose (hereinafter also referred to as HPMC), methyl cellulose, carboxymethyl cellulose sodium etc., sodium polyacrylate, polyvinyl alcohol, sodium alginate, guar gum etc.] and the like.

Such "basic inorganic salts" include, for example, basic inorganic salts of sodium, potassium, magnesium and/or calcium. Preferred are basic inorganic salts of magnesium and/or calcium. More preferred are basic inorganic salts of magnesium. Such basic inorganic salts of sodium include, for example, sodium carbonate, sodium hydrogen carbonate, disodium hydrogenphosphate and the like. Such basic inorganic salts of potassium include, for example, potassium carbonate, potassium hydrogen carbonate and the like. Such basic inorganic salts of magnesium include, for example, heavy magnesium carbonate, magnesium carbonate, magnesium oxide, magnesium hydroxide, magnesium metasilicate aluminate, magnesium silicate, magnesium aluminate, synthetic hydrotalcite [Mg₆Al₂ (OH)₁₆·CO₃·4H₂O] , and alumina hydroxide magnesium. Preferred are heavy magnesium carbonate, magnesium carbonate, magnesium oxide, magnesium hydroxide and the like. Such basic inorganic salts of calcium include, for example, precipitated calcium carbonate, calcium hydroxide, and the like.

Such "solvents" include, for example, water for injection, alcohol, propylene glycol, macrogol, sesame oil, corn oil, olive oil and the like.

Such "dissolution aids" include, for example, polyethylene glycol, propylene glycol, D-mannitol, benzyl benzoate, ethanol, trisaminomethane, cholesterol, triethanolamine, sodium carbonate, sodium citrate and the like.

Such "suspending agents" include, for example, surfactants such as stearyltriethanolamine, sodium lauryl sulfate, laurylaminopropionic acid, lecithin, benzalkonium chloride, benzethonium chloride, monostearic glycerol etc.; hydrophilic polymers such as polyvinyl alcohol, polyvinylpyrrolidone, carboxymethyl cellulose sodium, methyl cellulose, hydroxymethyl cellulose, hydroxyethyl cellulose, hydroxypropyl cellulose etc., and the like.

Such "isotonizing agents" include, for example, glucose, D-sorbitol, sodium chloride, glycerol, D-mannitol and the like.

Such "buffers" include, for example, buffer solutions of phosphates, acetates, carbonates, citrates etc., and the like.

Such "soothing agents" include, for example, benzyl alcohol and the like.

Such "preservatives" include, for example, p-oxybenzoic acid esters, chlorobutanol, benzyl alcohol, phenethyl alcohol, dehydroacetic acid, sorbic acid and the like.

Such "antioxidants" include, for example, sulfites, ascorbic acid, α-tocopherol and the like.

Such "coloring agents" include, for example, food colors such as Food Color Yellow No. 5, Food Color Red No. 2, Food Color Blue No. 2 etc.; food lake colors, red oxide and the like.

Such "sweetening agents" include, for example, saccharin sodium, dipotassium glycyrrhizinate, aspartame, stevia, thaumatin and the like.

Such "souring agents" include, for example, citric acid (citric anhydride), tartaric acid, malic acid and the like.

Such "bubbling agents" include, for example, sodium bicarbonate and the like.

Such "flavors" may be synthetic substances or naturally occurring substances, and include, for example, lemon, lime, orange, menthol, strawberry and the like.

The compound of the present invention may be prepared as a preparation for oral administration in accordance with a commonly-known method, by, for example, compression-shaping in the presence of a carrier such as an excipient, a disintegrant, a binder, a lubricant, or the like, and subsequently coating the preparation as necessary by a commonly known method for the purpose of taste masking, enteric dissolution or sustained release. For an enteric preparation, an intermediate layer may be provided by a commonly known method between the enteric layer and the drug-containing layer for the purpose of separation of the two layers.

For preparing the compound (I) or a salt thereof of the present invention as an orally disintegrating tablet, available methods include, for example, a method in which a core containing crystalline cellulose and lactose is coated with the compound (I) or a salt thereof of the present invention and, where necessary, a basic inorganic salt, and then further coated with a coating layer containing a water-soluble polymer to give a composition, which is coated with an enteric coating layer containing polyethylene glycol, further coated with an enteric coating layer containing triethyl citrate, still further coated with an enteric coating layer containing polyethylene glycol, and finally coated with mannitol to give fine granules, which are mixed with additives and shaped.

The above-mentioned "enteric coating layer" includes, for example, a layer consisting of a mixture of one or more kinds from aqueous enteric polymer substrates such as cellulose acetate phthalate (CAP), hydroxypropylmethyl cellulose phthalate, hydroxymethyl cellulose acetate succinate, methacrylic acid copolymers (e.g., Eudragit L30D-55 (trade name; produced by Rohm), Colicoat MAE30DP (trade name; produced by BASF), Polyquid PA30 (trade name; produced by Sanyo Chemical) etc.), carboxymethylethyl cellulose, shellac and the like; sustained-release substrates such as methacrylic acid copolymers (e.g., Eudragit NE30D (trade name), Eudragit RL30D (trade name), Eudragit RS30D (trade name) etc.) and the like; water-soluble polymers; plasticizers such as triethyl citrate, polyethylene glycol, acetylated monoglycerides, triacetin, castor oil and the like; and the like.

The above-mentioned "additive" includes, for example, water-soluble sugar alcohols (e.g., sorbitol, mannitol, maltitol, reduced starch saccharides, xylitol, reduced palatinose, erythritol etc.), crystalline cellulose (e.g., Ceolas KG 801, Avicel PH 101, Avicel PH 102, Avicel PH 301, Avicel PH 302, Avicel RC-591 (crystalline cellulose carmellose sodium) etc.), low-substituted hydroxypropyl cellulose (e.g., LH-22, LH-32, LH-23, LH-33 (Shin-Etsu Chemical), mixtures thereof etc.) and the like. Furthermore, binders, souring agents, bubbling agents, sweetening agents, flavors, lubricants, coloring agents, stabilizers, excipients, disintegrants etc. are also used.

The compound of the present invention may be further used in combination with 1 to 3 other active ingredients.

Such "other active ingredients" include, for example, anti-*Helicobacter pylori* active substances, imidazole compounds, bismuth salts, quinolone compounds, and so forth.

Such "anti-*Helicobacter pylori* active substances" include, for example, antibiotic penicillins (e.g., amoxicillin, benzylpenicillin, piperacillin, mecillinam etc.), antibiotic cefems (e.g., cefixime, cefaclor etc.), antibiotic macrolides (e.g., erythromycin, clarithromycin etc.), antibiotic tetracyclines (e.g., tetracycline, minocycline, streptomycin etc.), antibiotic aminoglycosides (e.g., gentamicin, amikacin etc.), imipenem and so forth. Of these substances, preferred are antibiotic penicillins, antibiotic macrolides and the like.

Such "imidazole compounds" include, for example, metronidazole, miconazole and the like.

Such "bismuth salts" include, for example, bismuth acetate, bismuth citrate and the like.

Such "quinolone compounds" include, for example, ofloxacin, ciploxacin and the like.

For eradication of *Helicobacter pylori*, a combination therapy of compound (I) or a salt thereof of the present invention with antibiotic penicillin (e.g., amoxicillin etc.) and antibiotic erythromycin (e.g., clarithromycin etc.) is particularly preferable.

Such "other active ingredients" and the compound (I) or a salt thereof of the present invention may be mixed, prepared as a single pharmaceutical composition [e.g., tablets, powders, granules, capsules (including soft capsules), liquids, injectable preparations, suppositories, sustained-release preparations etc.] in accordance with a commonly known method, and used in combination, and may also be prepared as separate preparations and administered to the same subject simultaneously or at a time interval.

### Examples

The present invention is explained in more detail by referring to Reference Examples and Examples, which are not to be construed as limitative.

In the following Reference Examples and Examples, the term "room temperature" indicates about 15 to 30°C.

¹H-NMR spectra were determined with CDCl₃ as the solvent using Varian Gemini-200 and Mercury-300; data are shown in chemical shift δ(ppm) from the internal standard tetramethylsilane.

Optical rotation [α]_{D} was determined at 20°C, using the DIP-370 Digital polarimeter (produced by JASCO).

The enantiomer excess (%ee) was measured by high performance liquid chromatography using an optically active column under the following conditions.
high performance liquid chromatography conditions (A);
column: CHIRALCEL OD (manufactured by Daicel Chemical Industries, Ltd.)
mobile phase: hexane/ethanol=90/10
flow rate: 1.0 mL/min.
detection: UV 285 nm
The other symbols used herein have the following definitions:
s: singlet
d: doublet
t: triplet
q: quartet
m: multiplet
bs: broad singlet
J: coupling constant

### Reference Example 1

### (R)-2-[[[3-Methyl-4-(2,2,2-trifluoroethoxy)-2-pyridinyl]methyl]sulfinyl]-1H-benzimidazole

2-[[[3-Methyl-4-(2,2,2-trifluoroethoxy)-2-pyridinyl]methyl]thio]-1H-benzimidazole (4.5 kg, 12.7 mol, containing water 1.89 g), toluene (22 L), water (25 g, 1.39 mol, 1.49 mol as total water content) and (+)-diethyl tartrate (0.958 L, 5.60 mol) were mixed under a nitrogen stream. Titanium(IV) isopropoxide (0.747 L, 2.53 mol) was added to the mixture at 50 - 60°C under a nitrogen stream, and the mixture was stirred at the same temperature for 30 min. Diisopropylethylamine (0.733 L, 4.44 mol) was added to the obtained mixture at room temperature under a nitrogen stream, and cumene hydroperoxide (6.88 L, content 82%, 37.5 mol) was added at -5°C to 5°C. The mixture was stirred at -5°C to 5°C for 1.5 hrs. 30% Aqueous sodium thiosulfate solution (17 L) was added to the reaction mixture under a nitrogen stream, and the remaining cumene hydroperoxide was decomposed. After partitioning, water (4.5 L), heptane (13.5 L), t-butyl methyl ether (18 L) and heptane (27 L) were added successively to the obtained organic layer, and crystals were precipitated under stirring. The crystals were separated and washed with t-butyl methyl ether-toluene (t-butyl methyl ether:toluene=4:1) (4 L). A suspension of the wet crystals in acetone (20 L) was added dropwise with stirring to a mixture of acetone (7 L) and water (34 L), and water (47 L) was added. The precipitated crystals were separated and washed with acetone-water (acetone:water=1:3)(4 L) and water (12 L). The wet crystals were dissolved in ethyl acetate (45 L) and water (3 L), and the mixture was partitioned. The trace amount of the insoluble material in the organic layer was filtered off, and triethylamine (0.2 L) was added. The mixture was concentrated under reduced pressure until the liquid amount became about 7 L. Methanol (2.3 L), ca. 12.5% aqueous ammonia (23 L, ca. 50°C) and t-butyl methyl ether (22 L, ca. 50°C) were added to the concentrate, and the mixture was partitioned. To the organic layer was added ca. 12.5% aqueous ammonia (11 L), and the mixture was partitioned (this step was repeated once). The aqueous layers were combined, and ethyl acetate (22 L) was added. Acetic acid was added dropwise under cooling, and pH was adjusted to about 8. After partitioning, the aqueous layer was extracted with ethyl acetate (11 L). The organic layers were combined and washed with ca. 20% brine (11 L). After addition of triethylamine (0.2 L), the organic layer was concentrated under reduced pressure. Acetone (5 L) was added to the concentrate, and the mixture was concentrated under reduced pressure. The concentrate was dissolved in acetone (9 L), and the solution was added dropwise to a mixture of acetone (4.5 L) and water (22.5 L), and water (18 L) was added dropwise to the obtained mixture. The precipitated crystals were separated and washed successively with cooled acetone-water (acetone:water=1:3) (3 L) and water (12 L). The wet crystals were dissolved in ethyl acetate (32 L). The aqueous layer thus separated was taken by partitioning, and the obtained organic layer was concentrated under reduced pressure until the liquid amount became about 14 L. Ethyl acetate (36 L) and activated carbon (270 g) were added to the residual liquid and, after stirring, the activated carbon was filtered off. The filtrate was concentrated under reduced pressure until the liquid amount became about 14 L. Heptane (90 L) was added dropwise to the remaining liquid at about 40°C. After stirring at the same temperature for about 30 min., the crystals were separated and washed with ethyl acetate-heptane (ethyl acetate:heptane=1:8, 6 L, ca. 40°C). Drying gave the title compound (3.4 kg). The enantiomer excess of this compound was 100% ee.

### Reference Example 2

### 1-Chloroethyl ethyl carbonate

1-Chloroethyl chloroformate (7.14 g) was added dropwise to a solution (60 mL) of ethanol (2.30 g) and pyridine (3.95 g) in tetrahydrofuran at -78°C. The mixture was stirred at room temperature for 2 days, and the precipitated solid was filtered off and washed with diethyl ether. The filtrate and the washing solution were combined, and the mixture was washed with saturated brine (15 mL) and dried over anhydrous magnesium sulfate. After filtration, the filtrate was concentrated under reduced pressure, and the residue was distilled under reduced pressure to give the title compound (7.01 g) as a colorless liquid.
¹H-NMR(CDCl₃): 1.34(3H,t,J=7.2Hz), 1.83(3H,d,J=5.8Hz), 4.27(2H,q,J=7.2Hz), 6.44(1H,q,J=5.8Hz).

### Reference Example 3

### 1-Chloroethyl trimethylacetate

To a mixture of trimethylacetyl chloride (12.1 g) and para-aldehyde (6.17 g) was added a catalytic amount of zinc chloride and the mixture was stirred at 90°C for 1 hr. Diethyl ether (150 mL) was added, and the mixture was washed with aqueous sodium hydrogen carbonate (100 mL) and dried over anhydrous magnesium sulfate. After concentration under reduced pressure, the residue was distilled under reduced pressure to give 1-chloroethyl trimethylacetate (12.2 g) as a colorless oil.
¹H-NMR(CDCl₃): 1.22(9H,s), 1.80(3H,d,J=5.9Hz), 6.54(1H,q, J=5.9Hz).

### Reference Example 4

### 1-Chloroethyl 2-methylpropanoate

To a mixture of isobutyryl chloride (10.7 g) and para-aldehyde (6.17 g) was added a catalytic amount of zinc chloride and the mixture was stirred at 80°C for 1 hr. Diethyl ether (150 mL) was added, and the mixture was washed with aqueous sodium hydrogen carbonate (100 mL) and dried over anhydrous magnesium sulfate. After concentration under reduced pressure, the residue was distilled under reduced pressure to give 1-chloroethyl 2-methylpropanoate (8.17 g) as a colorless oil.
¹H-NMR (CDCl₃) : 1.19(3H,d,J=7.0Hz), 1.20(3H,d,J=7.0Hz), 1.79(3H,d,J=5.9Hz), 2.47-2.69(1H,m), 6.55(1H,q,J=5.9Hz).

### Reference Example 5

### 1-Chloropropyl trimethylacetate

To a mixture of trimethylacetyl chloride (12.1 g) and propionaldehyde (8.13 g) was added a catalytic amount of zinc chloride and the mixture was stirred at 90°C for 30 min. Diethyl ether (200 mL) was added, and the mixture was washed with aqueous sodium hydrogen carbonate (100 mL) and dried over anhydrous magnesium sulfate. After concentration under reduced pressure, the residue was distilled under reduced pressure to give 1-chloropropyl trimethylacetate (9.13 g) as a pale-yellow oil.
¹H-NMR (CDCl₃) : 1.05(3H,t,J=7.3Hz), 1.23 (9H,s), 1.97-2.13 (2H,m), 6.38 (1H,t,J=5.7Hz).

### Reference Example 6

### 1-Chloroethyl 1,3-diethoxy-2-propyl carbonate

Using 1,3-diethoxy-2-propanol (5.00 g) and by a similar operation as in Reference Example 2, the title compound (8.03 g) was obtained as a pale-yellow liquid.
¹H-NMR(CDCl₃): 1.10-1.30(6H,m), 1.83(3H,d,J=5.6Hz), 3.40-3.70(8H,m), 5.02 (1H, quintet, J=5.2Hz) , 6.43(1H,q,J=5.6Hz).

### Reference Example 7

### 1,3-Diethoxy-2-propyl 1-iodoethyl carbonate

To a solution (5 mL) of the compound (3.0 g) of Reference Example 6 in carbon disulfide were added sodium iodide (2.39 g) and zinc chloride (0.15 g) and the mixture was stirred at room temperature for 2 hrs. The reaction solution was extracted with diethyl ether - water, and the organic layer was washed successively with saturated brine, aqueous sodium thiosulfate solution and saturated brine. After drying over anhydrous sodium sulfate, and the solvent was evaporated under reduced pressure to give the title compound (3.15 g) as a pale-yellow liquid.
¹H-NMR (CDCl₃) : 1.10-1.40 (6H,m), 2.24(3H,d,J=6.2Hz), 3.40-3.80(8H,m), 5.02(1H,m), 6.77(1H,q,J=6.2Hz).

### Reference Example 8

### 1-Chloroethyl 1,3-dimethoxy-2-propyl carbonate

Using 1,3-dimethoxy-2-propanol (5.00 g) and by a similar operation as in Reference Example 2, the title compound (8.08 g) was obtained as a pale-yellow liquid.
¹H-NMR(CDCl₃): 1.83(3H,d,J=5.6Hz), 3.38(6H,s), 3.59(4H,d,J=5.2Hz), 5.03 (1H, quint, J=5.2Hz), 6.43(1H,q,J=5.6Hz).

### Reference Example 9

### 1,3-Dimethoxy-2-propyl 1-iodoethyl carbonate

Using the compound (3.00 g) of Reference Example 8 and by a similar operation as in Reference Example 7, the title compound (3.45 g) was obtained as a colorless liquid.
¹H-NMR (CDCl₃) : 2.24(3H,d,J=5.8Hz). 3.38(3H,s), 3.39(3H,s), 3.55-3.61(4H,m), 4.95-5.10 (1H,m) , 6.76(1H,q,J=5.8Hz).

### Reference Example 10

### 1-Chloroethyl tetrahydro-2H-pyran-4-yl carbonate

Using 4-hydroxytetrahydropyran (4.00 g) and by a similar operation as in Reference Example 2, the title compound (6.92 g) was obtained as a pale-yellow liquid.
¹H-NMR(CDCl₃): 1.65-1.90(5H,m), 1.93-2.10(2H,m), 3.45-3.61(2H,m), 3.88-4.00(2H,m), 4.80-5.00(1H,m), 6.43(1H,q, J=5.8Hz).

### Reference Example 11

### Tetrahydro-2H-pyran-4-yl 1-iodoethyl carbonate

Using the compound (3.00 g) of Reference Example 10 and by a similar operation as in Reference Example 7, the title compound (3.02 g) was obtained as a colorless liquid.
¹H-NMR(CDCl₃): 1.60-1.90(2H,m), 1.95-2.10 (2H,m). 2.25(3H,d,J=5.8Hz), 3.45-3.65(2H,m), 3.85-4.00(2H,m), 4.80-5.00(1H,m), 6.76(1H,q,J=5.8Hz).

### Reference Example 12

### 1-Chloroethyl 2-methoxyethyl carbonate

Using 2-methoxyethanol (6.00 g) and by a similar operation as in Reference Example 2, the title compound (6.73 g) was obtained as a pale-yellow liquid.
¹H-NMR(CDCl₃): 1.83(3H,d,J=6.0Hz), 3.40(3H,s), 3.64(2H,t,J=4.6Hz), 4.30-4.40(2H,m), 6.42(1H,q,J=5.8Hz).

### Reference Example 13

### 2-Methoxyethyl 1-iodoethyl carbonate

Using the compound (3.00 g) of Reference Example 12 and by a similar operation as in Reference Example 7, the title compound (3.70 g) was obtained as a colorless liquid.
¹H-NMR (CDCl₃) : 2.24(3H,d,J=6.0Hz), 3.40(3H,s), 3.61(2H,t,J=4.6Hz), 4.30-4.40(2H,m), 6.76(1H,q,J-6.0Hz).

### Reference Example 14

### 2,2-Bis(methoxymethyl)propanoic acid

To a solution (100 mL) of 2,2-bis(hydroxymethyl)propanoic acid (10.0 g) in N,N-dimethylformamide was added sodium hydride (6.27 g) under ice-cooling. After generation of hydrogen was not observed, iodomethane (16.2 mL) was added and the mixture was stirred at room temperature for 18 hrs. 6N Aqueous sodium hydroxide solution (12.4 mL) was added to the reaction solution and the mixture was stirred for 3 hrs. The mixture was concentrated under reduced pressure. To the residue was added conc. hydrochloric acid and the obtained aqueous acidic solution was extracted with diethyl ether. The extract was washed with saturated brine, dried over anhydrous sodium sulfate and concentrated under reduced pressure to give the title compound (7.50 g) as a pale-yellow oil.
¹H-NMR (CDCl₃) : 1.22(3H,s), 3.36(6H,s), 3.51(4H,s), 6.50-7.30(1H,br).

### Reference Example 15

### 1-Iodoethyl 2,2-bis(methoxymethyl)propanoate

The compound (2.0 g) of Reference Example 14 was dissolved in thionyl chloride (5.0 mL) and the mixture was stirred at room temperature for 1 hr. The reaction solution was concentrated under reduced pressure and toluene (10 mL) was added to the residue. Insoluble materials were filtered off and the filtrate was concentrated under reduced pressure. To the obtained colorless oil were added para-aldehyde (0.812 g) and zinc chloride (10 mg) and the mixture was stirred at 50°C for 1 hr. The reaction solution was extracted with ethyl acetate - water, and the organic layer was washed with saturated brine, dried over anhydrous magnesium sulfate and concentrated under reduced pressure to give 1-chloroethyl 2,2-bis(methoxymethyl)propanoate(1.32 g) as a pale-yellow oil. Using 1-chloroethyl 2,2-bis(methoxymethyl)propanoate(1.30 g) and by a similar operation as in Reference Example 7, the title compound (1.20 g) was obtained as a colorless liquid.
¹H-NMR(CDCl₃): 1.18(3H,s), 2.20(3H,d,J=5.8Hz), 3.34(6H,s), 3.48(4H,s), 6.90(1H,q,J=5.8Hz).

### Reference Example 16

### 1-Iodoethyl cyclopentanecarboxylate

To a solution (30 mL) of sodium iodide (5.09 g) and para-aldehyde (1.10g) in acetonitrile was added cyclopentanecarbonyl chloride (3.0 g) under ice-cooling, and the mixture was stirred for 30 min. The reaction solution was extracted with ethyl acetate - water, and the organic layer was washed with saturated brine, dried over anhydrous magnesium sulfate and concentrated under reduced pressure to give the title compound (4.64 g) as a pale-yellow oil.
¹H-NMR (CDCl₃) : 1.40-2.00(8H,m), 2.20(3H,d,J=6.2Hz), 2.65-2.85(1H,m), 6.86(1H,q,J=6.2Hz).

### Reference Example 17

### 1-Chloroethyl cyclopentyl carbonate

A solution (20 mL) of 1-chloroethyl chloroformate (3.84 mL) in tetrahydrofuran (20 mL) was added dropwise to a solution of cyclopentanol (3.06 mL) and pyridine (5.46 mL) in tetrahydrofuran (100 mL) at 0°C. The mixture was stirred at room temperature for 24 hrs. and the precipitated solid was filtered off. Ethyl acetate (100 mL) was added to the filtrate and the mixture was washed with 1N hydrochloric acid (100 mL) and saturated brine (100mLx2) and dried over anhydrous sodium sulfate. After filtration, it was concentrated under reduced pressure to give the title compound (5.44 g) as a colorless oil.
¹H-NMR(CDCl₃) : 1.43-1.97(8H,m), 1.83(3H,d,J=6.0Hz), 5.13-5.19(1H,m), 6.43(1H,q,J=6.0Hz).

### Reference Example 18

### Cyclopentyl 1-iodoethyl carbonate

To a solution of 1-chloroethyl cyclopentyl carbonate in carbon disulfide (15 mL) were successively added sodium iodide (5.93 g) and zinc chloride (216 mg) and the mixture was stirred at room temperature for 4 hrs. Diethyl ether (100 mL) was added to the reaction solution and the mixture was washed with water (50 mL), 5% aqueous sodium thiosulfate solution (50 mL) and saturated brine (50 mL) and dried over anhydrous sodium sulfate. After filtration, it was concentrated under reduced pressure to give the title compound (6.59 g) as a pale-yellow oil.
¹H-NMR(CDCl₃): 1.56-2.09(8H,m), 2.24(3H,d,J=6.2Hz), 5.12-5.20(1H,m), 6.76(1H,q,J=6.2Hz).

### Reference Example 19

### 2-(Acetylamino)ethyl 1-chloroethyl carbonate

A solution of 1-chloroethyl chloroformate (3.92 mL) in tetrahydrofuran (20 mL) was added dropwise to a solution of N-acetylethanolamine (3.10 mL) and pyridine (5.46 mL) in tetrahydrofuran (100 mL) at 0°C. The mixture was stirred at room temperature for 24 hrs. and the precipitated solid was filtered off. Ethyl acetate (100 mL) was added to the filtrate and the mixture was washed with 1N hydrochloric acid (100 mL) and saturated brine (100mL×2) and dried over anhydrous sodium sulfate. After filtration, it was concentrated under reduced pressure to give the title compound (6.05 g) as a colorless oil.
¹H-NMR (CDCl₃) : 1.84(3H,d,J-5.8Hz), 2.02(3H,s), 3.60(2H,q,J=5.6Hz), 4.18-4.39(2H,m), 6.27(1H,brs), 6.86(1H,q,J=5.8Hz).

### Reference Example 20

### 2-(Acetylamino)ethyl 1-iodoethyl carbonate

To a solution of 2-(acetylamino)ethyl 1-chloroethyl carbonate (3.0 g) in acetonitrile (100 mL) was added sodium iodide (21.5 g) and the mixture was stirred under an argon atmosphere at 70°C for 2 hrs. The precipitated solid was filtered off and ethyl acetate (100 mL) was added. The mixture was washed with 5% aqueous sodium thiosulfate solution (100 mL) and saturated brine (100 mL) and dried over anhydrous sodium sulfate. After filtration, it was concentrated under reduced pressure to give the title compound (1.64 g) as a pale-yellow oil.
¹H-NMR(CDCl₃): 2.04(3H,s), 2.26(3H,d,J=6.3Hz), 3.58(2H,d,J=5.7Hz), 4.24-4.37(2H,m), 6.24(1H,bs), 6.76 (1H,q,J=6.3Hz).

### Reference Example 21

### Diethyl 3-[[(2-chloroethoxy)carbonyl]oxy]pentanedioate

A solution of 1-chloroethyl chloroformate (3.92 mL) in tetrahydrofuran (20 mL) was added dropwise to a solution of diethyl 3-hydroxyglutarate (6.24 mL) and pyridine (5.46 mL) in tetrahydrofuran (100 mL) at 0°C. The mixture was stirred at room temperature for 24 hrs. and the precipitated solid was filtered off. Ethyl acetate (100 mL) was added to the filtrate and the mixture was washed with water (100 mL), 1N hydrochloric acid (100 mL) and saturated brine (100 mL) and dried over anhydrous sodium sulfate. After filtration, it was concentrated under reduced pressure to give the title compound (9.68 g) as a colorless oil.
¹H-NMR (CDCl₃) : 1.26(3H,t,J=7.0Hz), 1.27(3H,t,J=7.0Hz), 1.83(3H,d,J=6.0Hz), 2.78(4H,d,J=6.6Hz), 4.16(2H,q,J=7.0Hz), 4.17(2H,q,J=7.0Hz), 5.48(1H,quintet,J=6.6Hz), 6.42(1H,q,J=6.0Hz).

### Reference Example 22

### Diethyl 3-[[(1-iodoethoxy)carbonyl]oxy]pentadioate

To a solution of diethyl 3-[[(1-chloroethoxy)carbonyl]oxy]pentanedioate (3 g) in acetonitrile(100 mL) was added sodium iodide (21.5 g) and the mixture was stirred under an argon atmosphere at 70°C for 3 hrs. The precipitated solid was filtered off and ethyl acetate (100 mL) was added. The mixture was washed with water (100 mL), 5% aqueous sodium thiosulfate solution (100 mL) and saturated brine (100 mL) and dried over anhydrous sodium sulfate. After filtration, it was concentrated under reduced pressure to give the title compound (3.26 g) as a pale-yellow oil.
¹H-NMR (CDCl₃) : 1.23-1.31(6H,m), 2.23(3H,d,J=6.0Hz), 2.71-2.85(4H,m), 4.09-4.22(4H,m), 5.48(1H,quintet,J=6.0Hz), 6.76(1H, q, J=6.0Hz).

### Reference Example 23

### 1-Iodoethyl acetate

To a solution of para-aldehyde (1.45 g) and sodium iodide (4.95 g) in acetonitrile (30 mL) was added acetyl chloride (2.13 mL) under ice-cooling, and the mixture was stirred at 0°C for 2 hrs. The reaction solution was poured into ice water (100 mL) and the mixture was extracted with diethyl ether (50 mLx2). The extract was washed successively with water (100 mL), 5% aqueous sodium thiosulfate solution (100 mL), aqueous sodium hydrogen carbonate (100 mL) and saturated brine (100 mL) and dried over anhydrous sodium sulfate. After filtration, it was concentrated under reduced pressure to give the title compound (3.10 g) as a pale-yellow oil.
¹H-NMR(CDCl₃): 2.08(3H,s), 2.20(3H,d,J=6.2Hz), 6.83 (1H,q,J=4.2Hz).

### Reference Example 24

### 1-Chloroethyl 1,3-dioxan-5-yl carbonate

A solution of 1-chloroethyl chloroformate (3.84 mL) in tetrahydrofuran (20 mL) was added dropwise to a solution of glycerol formal (2.92 mL) and pyridine (5.46 mL) in tetrahydrofuran (100 mL) at 0°C. The mixture was stirred at room temperature for 24 hrs. and the precipitated solid was filtered off. Ethyl acetate (100 mL) was added to the filtrate and the mixture was washed with water (100 mL), 1N hydrochloric acid (100 mL) and saturated brine (100 mL) and dried over anhydrous sodium sulfate. After filtration, the filtrate was concentrated under reduced pressure, and the obtained residue was purified by silica gel flash column chromatography (eluted with ethyl acetate:hexane=1:40-1:10) to give the title compound (2.95 g) as a colorless oil.
¹H-NMR(CDCl₃): 1.85(3H,d,J=6.0Hz), 4.04(4H,t,J=3.0Hz), 4.66(1H,quintet,J=3.0Hz), 4.81(1H,d,J=6.0Hz), 4.95(1H,d,J=6.0Hz), 6.42(1H,q,J=6.0Hz).

### Reference Example 25

### 1,3-Dioxan-5-yl 1-iodoethyl carbonate

To a solution of 1-chloroethyl 1,3-dioxan-5-yl carbonate (2.95 g) in acetonitrile (100 mL) was added sodium iodide (21.0 g) and the mixture was stirred under an argon atmosphere at 70°C for 3 hrs. The precipitated solid was filtered off and ethyl acetate (100 mL) was added. The mixture was washed with water (100 mL), 5% aqueous sodium thiosulfate solution (100 mL) and saturated brine (100 mL) and dried over anhydrous sodium sulfate. After filtration, it was concentrated under reduced pressure to give the title compound (2.43 g) as a pale-yellow oil.
¹H-NMR(CDCl₃): 2.26(3H,d,J=6.0Hz), 4.03(4H,t,J=2.8Hz), 4.66(1H,quintet,J-2.8Hz), 4.81(1H,d,J=6.2Hz), 4.95(1H,d,J=6.2Hz), 6.75(1H,q,J=6.0Hz).

### Reference Example 26

### 1-Iodoethyl cyclohexanecarboxylate

To a solution (30 mL) of para-aldehyde (1.45 g) and sodium iodide (4.50 g) in acetonitrile was added dropwise cyclohexanecarbonyl chloride (4.40 g) under ice-cooling. After stirring under ice-cooling for 2 hrs., ice water, sodium thiosulfate, sodium hydrogen carbonate, sodium chloride and diethyl ether were added. The organic layer was separated, dried over anhydrous magnesium sulfate and concentrated under reduced pressure to give the title compound (6.84 g) as a dark red purple oil.
¹H-NMR (CDCl₃) : 1.20-2.00(10H,m), 2.19(3H,d,J=6.2Hz), 2.20-2.40(1H,m), 6.86(1H,q,J=6.2Hz).

### Reference Example 27

### 1-Iodoethyl methoxyacetate

To a solution (30 mL) of para-aldehyde (1.45 g) and sodium iodide (4.50 g) in acetonitrile was added dropwise methoxyacetyl chloride (3.26 g) under ice-cooling. After stirring under ice-cooling for 2.5 hrs., diisopropyl ether (100 mL) was added and the mixture was washed with aqueous sodium thiosulfate solution (50 mL), aqueous sodium hydrogen carbonate (50 mL) and brine (50 mL). The mixture was dried over anhydrous magnesium sulfate and concentrated under reduced pressure to give the title compound (2.02 g) as a dark red purple oil.
¹H-NMR(CDCl₃) : 2.22(3H,d,J=6.2Hz), 3.47(3H,s), 4.03(2H,s), 6.93(1H,q,J=6. 2Hz).

### Example 1

### 1-[2-[[[3-Methyl-4-(2,2,2-trifluoroethoxy)-2-pyridinyl]methyl]sulfinyl]-1H-benzimidazol-1-yl]ethyl benzoate

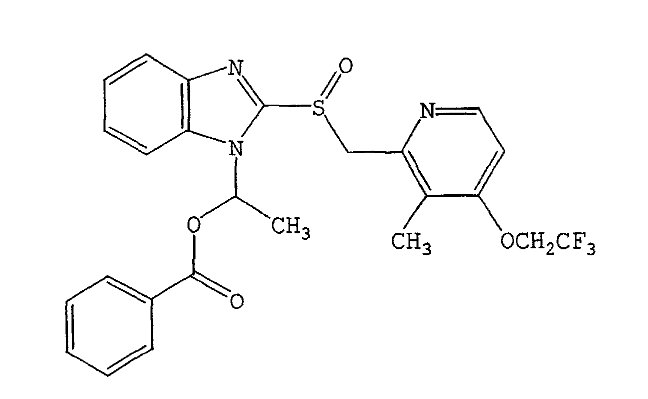

To a solution (50 mL) of 1-chloroethyl benzoate (2.22 g) and 2-[[[3-methyl-4-(2,2,2-trifluoroethoxy)-2-pyridinyl]methyl]thio]benzimidazole (2.12 g) in acetonitrile were added sodium iodide (0.45 g) and potassium carbonate (1.66 g) and the mixture was stirred at 60°C for 10 hrs. 1-Chloroethyl benzoate (1.11 g) was added and the mixture was stirred at 60°C for 12 hrs. After concentration under reduced pressure, ethyl acetate (200 mL) and saturated aqueous sodium hydrogen carbonate (100 mL) was added to the residue and the mixture was extracted. The ethyl acetate layer was separated and washed with 10% aqueous sodium sulfite solution (50 mL). It was dried over anhydrous magnesium sulfate and concentrated under reduced pressure. The residue was purified by silica gel chromatography (eluted with ethyl acetate:hexane=1:2-1:1) to give 1-[2-[[[3-methyl-4-(2,2,2-trifluoroethoxy)-2-pyridinyl]methyl]thio]-1H-benzimidazol-1-yl]ethyl benzoate (0.86 g) as a colorless solid.
¹H-NMR (CDCl₃): 1.97(3H,d,J=6.3Hz), 2.35(3H,s), 4.39(2H,q,J=7.8Hz), 4.85(1H,d,J=13.2Hz), 4.91(1H,d,J=13.2Hz), 6.65(1H,d,J=5.7Hz), 7.21-7.31(3H,m), 7.40-7.46(2H,m), 7.57(1H,t,J=7.4Hz), 7.64-7.72(2H,m), 8.04(2H,d,J=7.5Hz), 8.37(1H,d,J=5.7Hz).

To a solution (10 mL) of 1-[2-[[[3-methyl-4-(2,2,2-trifluoroethoxy)-2-pyridinyl]methyl]thio]-1H-benzimidazol-1-yl]ethyl benzoate (0.86 g) in dichloromethane was added dropwise a solution (10 mL) of 3-chloroperoxybenzoic acid (content: ca. 65%:0.455 g) in dichloromethane under ice-cooling. After stirring under ice-cooling for 1.5 hrs., the mixture was diluted with dichloromethane (30 mL) and washed with 10% aqueous sodium sulfite solution (20 mL) and saturated aqueous sodium hydrogen carbonate (20 mL). The mixture was dried over anhydrous magnesium sulfate and concentrated under reduced pressure. The residue was purified by silica gel chromatography (eluted with ethyl acetate:hexane=1:1) to give a diastereomeric mixture (0.64 g) of the title compound. Crystallization from ethanol, recrystallization from ethyl acetate - hexane, recrystallization from acetone-hexane, recrystallization from ethyl acetate - hexane and recrystallization from ethyl acetate-methanol gave a less polar diastereomer (0.29 g) of the title compound as a colorless solid.
¹H-NMR(CDCl₃): 2.12(3H,d,J=6.2Hz), 2.37(3H,s), 4.36(2H,q,J=7.8Hz), 5.12(2H,s), 6.61(1H,d,J=5.7Hz), 7.31-7.48(4H,m), 7.58-7.72(2H,m), 7.78-7.90(2H,m), 8.04(2H,d,J=7.4Hz), 8.34(1H,d,J=5.7Hz).

### Example 2

### Ethyl 1-[2-[[[3-methyl-4-(2,2,2-trifluoroethoxy)-2-pyridinyl]methyl]sulfinyl]-1H-benzimidazol-1-yl]ethyl carbonate

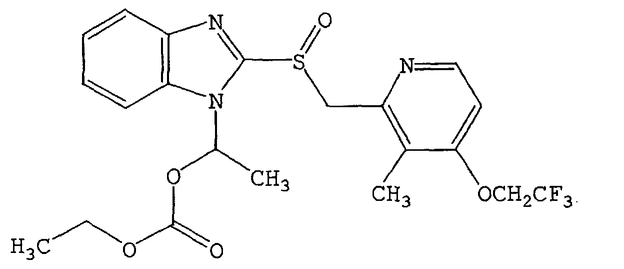

A solution (50 mL) of 1-chloroethyl ethyl carbonate (1.52 g) and sodium iodide (4.48 g) in acetonitrile was stirred at 60°C for 1 hr. Acetonitrile was evaporated under reduced pressure and the residue was extracted with diethyl ether (60 mL). After filtration, the filtrate was concentrated under reduced pressure. To the residue were added 2-[[[3-methyl-4-(2,2,2-trifluoroethoxy)-2-pyridinyl]methyl]thio]benzimidazole (1.57 g) and acetonitrile (30 mL) and the mixture was stirred at room temperature for 3 days. After concentration under reduced pressure, ethyl acetate (200 mL) and saturated aqueous sodium hydrogen carbonate (100 mL) were added to the residue to allow for extraction. The ethyl acetate layer was separated and washed with 10% aqueous sodium sulfite solution (50 mL) and saturated brine (50 mL). It was dried over anhydrous magnesium sulfate and concentrated under reduced pressure. The residue was purified by silica gel chromatography (eluted with ethyl acetate:hexane=1:2) to give ethyl 1-(2-[[[3-methyl-4-(2,2,2-trifluoroethoxy)-2-pyridinyl]methyl]thio]-1H-benzimidazol-1-yl]ethyl carbonate (0.84 g) as a colorless solid.
¹H-NMR (CDCl₃) : 1.25(3H,t,J=7.1Hz), 1.87(3H,d,J=6.3Hz), 2.35(3H,s), 4.05-4.25(2H,m), 4.40(2H,q,J=7.8Hz), 4.83(1H,d,J=13.5Hz), 4.88(1H,d,J=13.5Hz), 6.66(1H,d,J=5.4Hz), 7.19-7.28(2H,m), 7.60(1H,m), 7.70(1H,m), 8.36(1H,d,J=5.4Hz).

To a solution (10 mL) of ethyl 1-[2-[[[3-methyl-4-(2,2,2-trifluoroethoxy)-2-pyridinyl]methyl]thio]-1H-benzimidazol-1-yl]ethyl carbonate (0.84 g) in dichloromethane was added dropwise a solution (10 mL) of 3-chloroperoxybenzoic acid (content: ca. 65%: 0.475 g) in dichloromethane under ice-cooling. After stirring under ice-cooling for 1.5 hrs., the mixture was diluted with dichloromethane (30 mL) and washed with 10% aqueous sodium sulfite solution (20 mL) and saturated aqueous sodium hydrogen carbonate (20 mL). The mixture was dried over anhydrous magnesium sulfate and concentrated under reduced pressure. The residue was purified by silica gel chromatography (eluted with ethyl acetate:hexane=1:1) to give a diastereomeric mixture (0.64 g) of the title compound. Crystallization from diisopropyl ether and 2 repeats of recrystallization from ethyl acetate - hexane gave a less polar diastereomer (0.31 g) of the title compound as a colorless solid.
¹H-NMR (CDCl₃) : 1.24(3H,t,J=7.2Hz), 2.01(3H,d,J=6.3Hz), 2.38(3H,s), 4.03-4.24(2H,m), 4.40(2H,q,J=7.7Hz), 5.00(1H,d,J=13.7Hz), 5.12(1H,d,J=13.7Hz), 6.67(1H,d,J=5.7Hz), 7.32-7.43(3H,m), 7.76(1H,m), 7.87(1H,m), 8.39(1H,d,J=5.5Hz).

### Example 3

### 1-[2-[[[3-Methyl-4-(2,2,2-trifluoroethoxy)-2-pyridinyl]methyl]sulfinyl]-1H-benzimidazol-1-yl]ethyl trimethylacetate

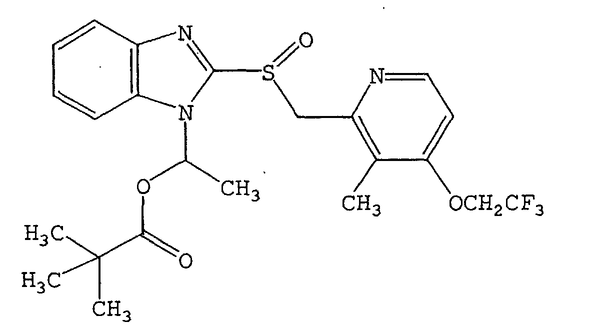

A mixture of 2-[[[3-methyl-4-(2,2,2-trifluoroethoxy)-2-pyridinyl]methyl]thio]benzimidazole (6.72 g), 1-chloroethyl trimethylacetate (6.59 g), sodium iodide (3.00 g), potassium carbonate (8.29 g) and acetonitrile (200 mL) was stirred at 60°C for 2 days. After concentration under reduced pressure, ethyl acetate (200 mL) was added to the reaction solution, and the mixture was washed with water (100 mL) and saturated brine (100 mL) and dried over anhydrous magnesium sulfate. After concentration under reduced pressure, the residue was purified by silica gel column chromatography (eluted with ethyl acetate:hexane=1:1 then 4:1) to give 1-[2-[[[3-methyl-4-(2,2,2-trifluoroethoxy)-2-pyridinyl]methyl]thio]-1H-benzimidazol-1-yl]ethyl trimethylacetate (5.19 g) as an orange oil.
¹H-NMR(CDCl₃): 1.16(9H,s), 1.81(3H,d,J=6.2Hz), 2.35(3H,s), 4.40(2H,q,J=7.8Hz), 4.86(2H,s), 6.65(1H,d,J=5.6Hz), 6.98(1H,q,J=6.2Hz), 7.15-7.30(2H,m), 7.50-7.60(1H,m), 7.65-7.75(1H,m), 8.36(1H,d,J=5.6Hz).

To a solution (120 mL) of 1-[2-[[[3-methyl-4-(2,2,2-trifluoroethoxy)-2-pyridinyl]methyl]thio]-1H-benzimidazol-1-yl]ethyl trimethylacetate (5.42 g) in dichloromethane was added 3-chloroperoxybenzoic acid (content: ca. 65%: 3.19 g) at - 25°C. The mixture was stirred at -25°C for 15 min. and at 0°C for 30 min., and the mixture was washed with aqueous sodium hydrogen carbonate (100 mL) and 10% aqueous sodium thiosulfate (50 mL) and dried over anhydrous sodium sulfate. After concentration under reduced pressure, the residue was purified by silica gel column chromatography (eluted with ethyl acetate:hexane=1:1, then successively with ethyl acetate). The obtained solid was washed with a mixed solution of ethyl acetate and diisopropyl ether and further recrystallized from ethyl acetate and diisopropyl ether to give a less polar diastereomer (2.13 g) of the title compound as a colorless solid.
¹H-NMR(CDCl₃): 1.14(9H,s), 1.96(3H,d,J=6.2Hz), 2.37(3H,s), 4.40(2H,q,J=7.9Hz), 5.03(1H,d,J=13.9Hz), 5.12(1H,d,J=13.9Hz), 6.66(1H,d,J=5.5Hz), 7.29-7.46(3H,m), 7.66-7.76(1H,m), 7.81-7.91(1H,m), 8.38(1H,d,J=5.5Hz).

### Example 4

### 1-[2-[[[3-Methyl-4-(2,2,2-trifluoroethoxy)-2-pyridinyl]methyl]sulfinyl]-1H-benzimidazol-1-yl]ethyl 2-methylpropanoate

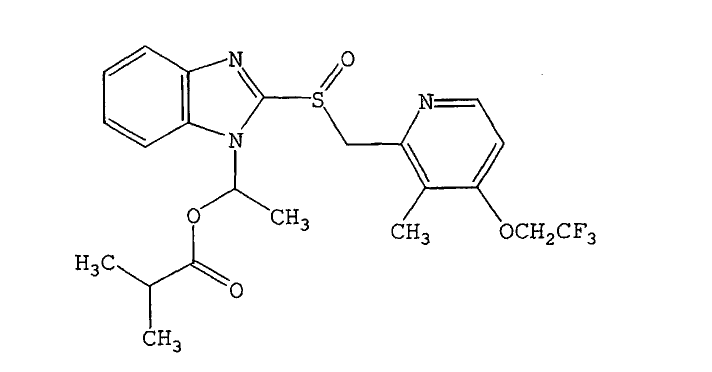

A mixture of 2-[[[3-methyl-4-(2,2,2-trifluoroethoxy)-2-pyridinyl]methyl]thio]benzimidazole (3.53 g), 1-chloroethyl 2-methylpropanoate (1.81 g), sodium iodide (3.00 g), potassium carbonate (2.76 g) and acetonitrile (100 mL) was stirred at 60°C for 10 hrs. 1-Chloroethyl 2-methylpropanoate (1.20 g) and potassium carbonate (1.38 g) were added and the mixture was stirred overnight at 60°C. The reaction solution was concentrated under reduced pressure and ethyl acetate (100 mL) was added. The mixture was washed with water (50 mL) and saturated brine (50 mL), and dried over anhydrous magnesium sulfate. After concentration under reduced pressure, the residue was purified by silica gel column chromatography (eluted with ethyl acetate:hexane=3:7 then 7:3) to give 1-[2-[[[3-methyl-4-(2,2,2-trifluoroethoxy)-2-pyridinyl]methyl]thio]-1H-benzimidazol-1-yl]ethyl 2-methylpropanoate (1.38 g) as a pale-yellow orange oil.
¹H-NMR (CDCl₃) : 1.09 (3H,d,J=6.9Hz), 1.15(3H,d,J=6.9Hz), 1.82(3H,d,J=6.2Hz), 2.35(3H,s), 2.48-2.66(1H,m), 4.40(2H,q,J=7.9Hz), 4.86(2H,s), 6.66(1H,d,J=5.6Hz), 7.00(1H,q,J=6.2Hz), 7.15-7.30(2H,m), 7.52-7.60(1H,m), 7.65-7.73(1H,m), 8.37(1H,d,J=5.6Hz).

To a solution (30 mL) of 1-[2-[[[3-methyl-4-(2,2,2-trifluoroethoxy)-2-pyridinyl]methyl]thio]-1H-benzimidazol-1-yl]ethyl 2-methylpropanoate (1.37 g) in dichloromethane was added 3-chloroperoxybenzoic acid (content: ca. 65%: 796 mg) at 0°C. The mixture was stirred at 0°C for 1 hr, washed with aqueous sodium hydrogen carbonate (30 mL) and 10% aqueous sodium thiosulfate (10 mL), and dried over anhydrous sodium sulfate. After concentration under reduced pressure, the residue was purified by silica gel column chromatography (eluted with ethyl acetate:hexane=1:1, then successively with ethyl acetate). The obtained solid was washed with a mixed solution of ethyl acetate and diisopropyl ether and further recrystallized from ethyl acetate and diisopropyl ether to give a less polar diastereomer (466 mg) of the title compound as a colorless solid.
¹H-NMR (CDCl₃) : 1.05 (3H,d,J=7.1Hz ) , 1.13 (3H,d,J=7.1Hz), 1.97(3H,d,J=6.2Hz), 2.38(3H,s), 2.47-2.69(1H,m), 4.40(2H,q,J=7.8Hz), 5.01(1H,d,J=13.8Hz), 5.12(1H,d,J=13.8Hz), 6.66(1H,d,J=5.3Hz), 7.30-7.46(3H,m), 7.67-7.78(1H,m), 7.82-7.92(1H,m), 8.38(1H,d,J=5.3Hz).

### Example 5

### 1-[2-[[[3-Methyl-4-(2,2,2-trifluoroethoxy)-2-pyridinyl]methyl]sulfinyl]-1H-benzimidazol-1-yl]propyl trimethylacetate

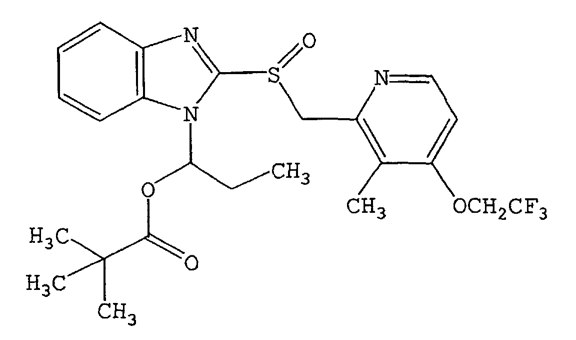

A mixture of 2-[[[3-methyl-4-(2,2,2-trifluoroethoxy)-2-pyridinyl]methyl]thio]benzimidazole (3.53 g), 1-chloropropyl trimethylacetate (1.78 g), sodium iodide (0.75 g), potassium carbonate (2.76 g) and acetonitrile (100 mL) was stirred overnight at 60°C. 1-Chloropropyl trimethylacetate (0.89 g) and potassium carbonate (0.69 g) were added and the mixture was stirred at 60°C for 5 hrs. 1-Chloropropyl trimethylacetate (0.89 g), sodium iodide (0.75 g) and potassium carbonate (0.69 g) were added and the mixture was stirred at 60°C for 6 hrs. 1-Chloropropyl trimethylacetate (1.78 g) and potassium carbonate (1.38 g) were added and the mixture was stirred at 60°C for 2 days. The reaction solution was concentrated under reduced pressure, and ethyl acetate (100 mL) was added. The mixture was washed with water (50 mL) and saturated brine (50 mL), and dried over anhydrous magnesium sulfate. After concentration under reduced pressure, the residue was purified by silica gel column chromatography (eluted with ethyl acetate:hexane=3:7 then 7:3) to give 1-[2-[[[3-methyl-4-(2,2,2-trifluoroethoxy)-2-pyridinyl]methyl]thio]-1H-benzimidazol-1-yl]propyl trimethylacetate (2.42 g) as an orange oil.
¹H-NMR (CDCl₃) : 0.87(3H,t,J=7.3Hz), 1.17(9H,s), 2.16-2.32(2H,m), 2.35(3H,s), 4.40(2H,q,J=7.8Hz), 4.87(2H,s), 6.65(1H,d,J=5.8Hz), 6.74(1H,t,J=7.4Hz), 7.14-7.29(2H,m), 7.48-7.59(1H,m), 7.64-7.74(1H,m), 8.36(1H,d,J=5.8Hz).

To a solution (50 mL) of 1-[2-[[[3-methyl-4-(2,2,2-trifluoroethoxy)-2-pyridinyl]methyl]thio]-1H-benzimidazol-1-yl]propyl trimethylacetate (2.31 g) in dichloromethane was added 3-chloroperoxybenzoic acid (content: ca. 65%: 1.33 g) at 0°C. The mixture was stirred at 0°C for 1 hr., and the mixture was washed with aqueous sodium hydrogen carbonate (30 mL) and 10% aqueous sodium thiosulfate (10 mL) and dried over anhydrous sodium sulfate. After concentration under reduced pressure, the residue was purified by silica gel column chromatography (eluted with ethyl acetate:hexane=1:1, then successively with ethyl acetate) and crystallized from ethyl acetate and diisopropyl ether. The crystals were collected by filtration, washed with diisopropyl ether and recrystallized from ethyl acetate and diisopropyl ether to give a less polar diastereomer (667 mg) of the title compound as a colorless solid.
¹H-NMR(CDCl₃) : 0.99(3H,t,J=7.5Hz), 1.15(9H,s), 2.22-2.52(2H,m), 2.37(3H,s), 4.40(2H,q,J=7.8Hz), 5.01(1H,d,J=13.9Hz), 5.14(1H,d,J=13.9Hz), 6.66(1H,d,J=5.5Hz), 7.10(1H,t,J=7.3Hz), 7.30-7.43(2H,m), 7.63-7.74(1H,m), 7.83-7.93(1H,m), 8.39(1H,d,J=5.5Hz).

### Example 6

### Cyclohexyl 1-[2-[[[3-methyl-4-(2,2,2-trifluoroethoxy)-2-pyridinyl]methyl]sulfinyl]-1H-benzimidazol-1-yl]ethyl carbonate

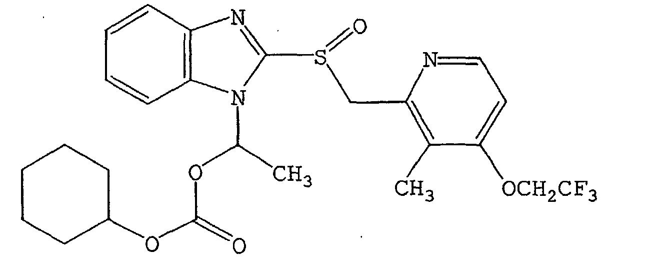

To a solution (50 mL) of 2-[[[3-methyl-4-(2,2,2-trifluoroethoxy)-2-pyridinyl]methyl]thio]benzimidazole (2.12 g) and 1-chloroethyl cyclohexylcarbonate (1.86 g) in acetonitrile were added sodium iodide (0.45 g) and potassium carbonate (1.66 g) and the mixture was stirred at 60°C for 34 hrs. Acetonitrile was evaporated under reduced pressure and the residue was extracted with ethyl acetate (150 mL) and water (50 mL). The organic layer was separated and washed with 10% aqueous sodium sulfite solution (30 mL). It was dried over anhydrous magnesium sulfate and concentrated under reduced pressure. The residue was purified by silica gel chromatography (eluted with ethyl acetate:hexane=1:1) to give cyclohexyl 1-[2-[[[3-methyl-4-(2,2,2-trifluoroethoxy)-2-pyridinyl]methyl]thio]-1H-benzimidazol-1-yl]ethyl carbonate (1.00 g) as an amorphous form.
¹H-NMR(CDCl₃): 1.08-1.98 (10H,m) , 1.86 (3H,d,J=6.5Hz) , 2.35 (3H, s), 4.44(2H,q,J=7.5Hz), 4.54(1H,m), 4.86(2H,s), 6.65(1H,d,J=5.8Hz), 6.86(1H,q,J=6.5Hz), 7.17-7.29(2H,m), 7.56-7.72 (2H,m) , 8.36(1H, d, J=5.8Hz).

To a solution (11 mL) of cyclohexyl 1-[2-[[[3-methyl-4-(2,2,2-trifluoroethoxy)-2-pyridinyl]methyl]thio]-1H-benzimidazol-1-yl]ethyl carbonate (0.99 g) in dichloromethane was added dropwise a solution (11 mL) of 3-chloroperoxybenzoic acid (content: ca. 65%: 0.505 g) in dichloromethane under ice-cooling. The mixture was stirred under ice-cooling for 2 hrs., and diluted with dichloromethane (30 mL). The mixture was washed with 10% aqueous sodium sulfite solution (20 mL) and saturated aqueous sodium hydrogen carbonate (20 mL), dried over anhydrous magnesium sulfate and concentrated under reduced pressure. The residue was purified by silica gel chromatography (eluted with ethyl acetate:hexane=1:1) and further purified by preparative HPLC (ODS-A manufactured by YMC Co., Ltd., diameter 20 mm, length 250 mm: eluted with acetonitrile:water=38:62, flow rate 20 mL/min) to give a more polar diastereomer (0.08 g) and a less polar diastereomer (0.13 g) of the title compound as colorless solid. More polar diastereomer
¹H-NMR (CDCl₃) : 1.18-2.02 (10H,m) , 1.92 (3H,d,J=6.2Hz), 2.26(3H,s), 4.38(2H,q,J=7.8Hz), 4.59(1H,m), 4.90(1H,d,J=14.0Hz), 5.30(1H,d,J=14.0Hz), 6.63(1H,d,J=5.9Hz), 7.24-7.41(2H,m)7.69(1H,q,J=6.2Hz), 7.70-7.82(2H,m), 8.31(1H,d,J=5.9Hz).
Less polar diastereomer
¹H-NMR (CDCl₃) : 1.10-1.99 (10H,m), 2.00 (3H,d,J=6.6Hz) , 2.38 (3H,s), 4.40(2H,q,J=7.8Hz), 4.51(1H,m), 4.99(1H,d,J=13.9Hz), 5.12(1H,d,J=13.9Hz), 6.67(1H,d,J=5.8Hz), 7.28-7.44(3H,m), 7.73-7.89(2H,m), 8.38(1H,d,J=5.8Hz).

### Example 7

### Cyclohexyl 1-[(R)-2-[[[3-methyl-4-(2,2,2-trifluoroethoxy)-2-pyridinyl]methyl]sulfinyl]-1H-benzimidazol-1-yl]ethyl carbonate

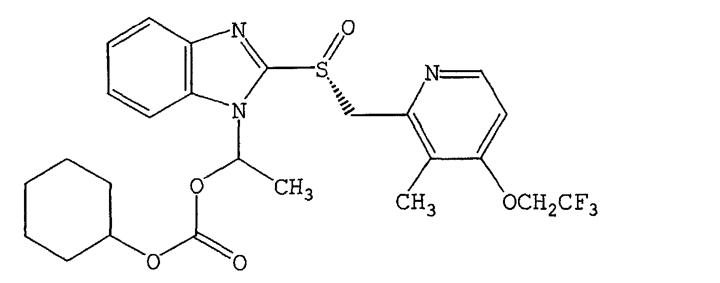

To a solution (25 mL) of 1-chloroethyl cyclohexylcarbonate (5.0 g) in carbon disulfide were added sodium iodide (6.8 g) and zinc chloride (0.25 g) and the mixture was stirred at room temperature for 3 hrs. The reaction solution was poured into ice water (100 mL) and the mixture was extracted twice with diethyl ether (50 mL). The organic layers were combined and washed with 10% aqueous sodium sulfite solution (30 mL), saturated aqueous sodium hydrogen carbonate (30 mL) and water (30 mL). The mixture was dried over anhydrous magnesium sulfate and concentrated under reduced pressure to give cyclohexyl 1-iodoethylcarbonate (6.10 g) as a liquid.

To a solution (100 mL) of (R)-2-[[[3-methyl-4-(2,2,2-trifluoroethoxy)-2-pyridinyl]methyl]sulfinyl]benzimidazole (4.43 g) and cyclohexyl 1-iodoethylcarbonate (5.46 g) in acetone was added cesium carbonate (7.82 g) and the mixture was stirred at 15°C for 2 hrs. Ethyl acetate (100 mL) was added, and after filtration, the filtrate was concentrated under reduced pressure. The residue was extracted with ethyl acetate (150 mL) and water (100 mL). The organic layer was separated, washed with 10% aqueous sodium sulfite solution (20 mL) and saturated brine (30 mL), dried over anhydrous magnesium sulfate, and concentrated under reduced pressure. The residue was purified by silica gel chromatography (eluted with acetonitrile:diisopropyl ether =1:4). It was further purified by preparative HPLC (ODS-A manufactured by YMC Co., Ltd., diameter 30 mm, length 250 mm: eluted with acetonitrile:water=38:62, flow rate 20 mL/min) to give a more polar diastereomer (1.65 g) and a less polar diastereomer (0.48 g) of the title compound as colorless solid.
More polar diastereomer
¹H-NMR(CDCl₃): 1.18-2.02(10H,m), 1.92(3H,d,J=6.2Hz), 2.26(3H,s), 4.38(2H,q,J=7.8Hz), 4.59(1H,m), 4.90(1H,d,J=14.0Hz), 5.30(1H,d,J=14.0Hz), 6.63(1H,d,J=5.9Hz), 7.24-7.41(2H,m)7.69(1H,q,J-6.2Hz), 7.70-7.82(2H,m), 8.31(1H,d,J=5.9Hz).
Less polar diastereomer
¹H-NMR (CDCl₃) : 1.10-1.99(10H,m), 2.00(3H,d,J=6.4Hz), 2.38(3H,s), 4.40(2H,q,J=7.9Hz), 4.51(1H,m), 5.00(1H,d,J=13.7Hz), 5.12(1H,d,J=13.7Hz), 6.67(1H,d,J=6.0Hz), 7.28-7.44(3H,m), 7.73-7.90(2H,m), 8.38(1H,d,J=6.0Hz).

### Example 8

### Ethyl 1-[(R)-2-[[[3-methyl-4-(2,2,2-trifluoroethoxy)-2-pyridinyl]methyl]sulfinyl]-1H-benzimidazal-1-yl]ethyl carbonate

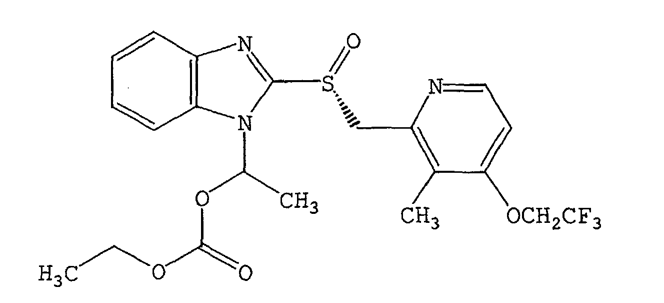

To a solution (12.5 mL) of 1-chloroethyl ethyl carbonate (2.50 g) in carbon disulfide were added sodium iodide (3.4 g) and zinc chloride (0.13 g) and the mixture was stirred at room temperature for 7 hrs. The reaction solution was poured into ice water (50 mL) and the mixture was extracted twice with diethyl ether (50 mL). The organic layers were combined and washed with 10% aqueous sodium sulfite solution (25 mL), saturated aqueous sodium hydrogen carbonate (25 mL) and water (25 mL). The mixture was dried over anhydrous magnesium sulfate and concentrated under reduced pressure to give ethyl 1-iodoethylcarbonate (3.53 g) as a liquid.

To a solution (50 mL) of (R)-2-[[[3-methyl-4-(2,2,2-trifluoroethoxy)-2-pyridinyl]methyl]sulfinyl]benzimidazole (3.70 g) and ethyl 1-iodoethylcarbonate (3.52 g) in acetone was added cesium carbonate (4.89 g) and the mixture was stirred at room temperature for 19 hrs. Acetone was evaporated under reduced pressure and the residue was extracted with ethyl acetate (150 mL) and water (100 mL). The organic layer was separated and washed with 10% aqueous sodium sulfite solution (50 mL) and saturated brine (50 mL). It was dried over anhydrous magnesium sulfate and concentrated under reduced pressure. The residue was purified by silica gel chromatography (eluted with ethyl acetate:hexane=1:1) and further purified by preparative HPLC (ODS-A manufactured by YMC Co., Ltd., diameter 30 mm, length 250 mm: eluted with acetonitrile:water=27:73, flow rate 20 mL/min) to give a more polar diastereomer (0.41 g) and a less polar diastereomer (0.67 g) of the title compound as colorless solid. More polar diastereomer
¹H-NMR (CDCl₃) : 1.27(3H,t,J=7.2Hz), 1.93(3H,d,J=6.3Hz), 2.26(3H,s), 4.08-4.28(2H,m), 4.38(2H,q,J=7.8Hz), 4.90(1H,d,J=13.7Hz), 5.31(1H,d,J=13.7Hz), 6.64(1H,d,J=6.0Hz), 7.28-7.42(2H,m), 7.69-7.83(3H,m), 8.32(1H,d,J=6.0Hz). Less polar diastereomer
¹H-NMR (CDCl₃) : 1.24(3H,t,J-7.2Hz), 2.01(3H,d,J=6.2Hz), 2.38(3H,s), 3.98-4.27(2H,m), 4.40(2H,q,J=7.8Hz), 4.99(1H,d,J=13.8Hz), 5.12(1H,d,J=13.8Hz), 6.67(1H,d,J=5.6Hz), 7.30-7.45(3H,m), 7.76(1H,m), 7.86(1H,m), 8.39(1H,d,J=5.6Hz).

### Example 9

### Benzyl 2-[2-[[[3-methyl-4-(2,2,2-trifluoroethoxy)-2-pyridinyl] methyl] sulfinyl]-1H-benzimidazol-1-yl] ethyl carbonate

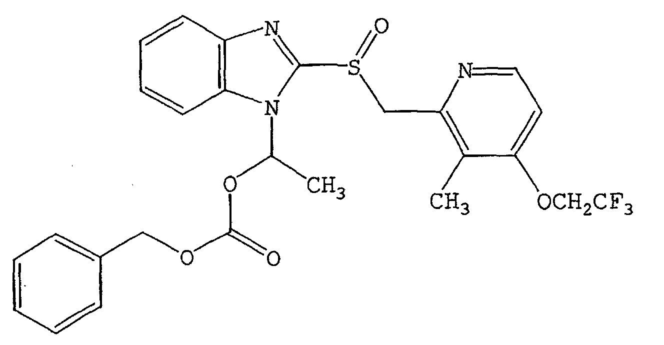

To a mixture of benzyl alcohol (5.17 mL), pyridine (4.85 mL) and dichloromethane (100 mL) was added 1-chloroethyl chloroformate (5.40 mL) at -78°C and the mixture was stirred at room temperature for 3 days. After concentration under reduced pressure, ethyl acetate (200 mL) was added to the reaction solution, and the mixture was washed with saturated brine (100 mL) and dried over anhydrous magnesium sulfate. It was concentrated under reduced pressure to give benzyl 1-chloroethyl carbonate (11.3 g) as a yellow oil.
¹H-NMR (CDCl₃) : 1.83(3H,d,J=5.8Hz), 5.20(1H,d,J=12.0Hz), 5.25(1H,d,J=12.0Hz), 6.44(1H,q,J=5.8Hz), 7.30-7.45(5H,m).

A mixture of benzyl 1-chloroethyl carbonate (2.15 g), sodium iodide (3.00 g), 18-crown-6 (132 mg) and toluene (20 mL) was stirred at 80°C for 12 hrs. and at room temperature for 2 days. Ethyl acetate (50 mL) was added to the reaction solution, and the mixture was washed with water (30 mL), 10% aqueous sodium thiosulfate (30 mL) and saturated brine (30 mL), dried over anhydrous magnesium sulfate and concentrated under reduced pressure. To the residue were added 2-[[[3-methyl-4-(2,2,2-trifluoroethoxy)-2-pyridinyl]methyl]thio]benzimidazole (3.53 g), sodium iodide (1.50 g), 18-crown-6 (264 mg), potassium carbonate (2.76 g) and acetonitrile (100 mL) and the mixture was stirred at 60°C for 18 hrs. After concentration under reduced pressure, ethyl acetate (100 mL) was added to the reaction solution. The mixture was washed with water (50 mL), 10% aqueous sodium thiosulfate (50 mL) and saturated brine (100 mL), and dried over anhydrous magnesium sulfate. After concentration under reduced pressure, the residue was purified by silica gel column chromatography (eluted with ethyl acetate:hexane=2:1, then successively with ethyl acetate). Diisopropyl ether was added to allow for solidification and the solid was collected by filtration to give benzyl 1-[2-[[[3-methyl-4-(2,2,2-trifluoroethoxy)-2-pyridinyl]methyl]thio]-1H-benzimidazol-1-yl] ethyl carbonate (703 mg) as a colorless solid.
¹H-NMR (CDCl₃) : 1.86(3H,d,J=6.5Hz), 2.35(3H,s), 4.40(2H,q,J=7.9Hz), 4.85(2H,s), 5.02(1H,d,J=11.7Hz), 5.17(1H,d,J=11.7Hz), 6.65(1H,d,J=5.5Hz), 6.90(1H,q,J=6.5Hz), 7.16-7.38(7H,m), 7.52-7.75(2H,m), 8.36(1H,d,J=5.5Hz).

To a solution (15 mL) of benzyl 1-[2-[[[3-methyl-4-(2,2,2-trifluoroethoxy)-2-pyridinyl]methyl]thio]-1H-benzimidazol-1-yl]ethyl carbonate (675 mg) in dichloromethane was added 3-chloroperoxybenzoic acid (content: ca. 65%: 372 mg) at 0°C and the mixture was stirred at 0°C for 3 hrs. The mixture was washed with aqueous sodium hydrogen carbonate (10 mL) and 10% aqueous sodium thiosulfate (10 mL) and dried over anhydrous sodium sulfate. After concentration under reduced pressure, the residue was purified by silica gel column chromatography (eluted with ethyl acetate:hexane=1:1, then successively with ethyl acetate). Ethyl acetate and diisopropyl ether were added to allow for solidification, and the solid was collected by filtration, washed and further recrystallized from ethyl acetate and diisopropyl ether to give a less polar diastereomer (320 mg) of the title compound as a colorless solid.
¹H-NMR (CDCl₃) : 1.97(3H,d,J=6.2Hz), 2.37(3H,s), 4.39(2H,q,J=8.0Hz), 4.96-5.19(4H,m), 6.63(1H,d,J=5.9Hz), 7.20-7.45(8H,m), 7.68-7.94(2H,m), 8.35(1H,d,J=5.9Hz).

### Example 10

### Isopropyl 1-[(R)-2-[[[3-methyl-4-(2,2,2-trifluoroethoxy)-2-pyridinyl]methyl]sulfinyl]-1H-benzimidazol-l-yl] ethyl carbonate

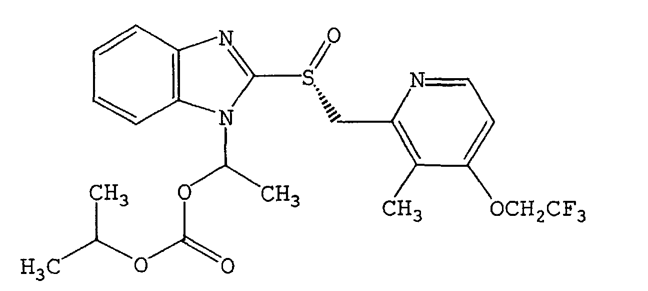

To a mixture of 2-propanol (3.83 mL), pyridine (4.85 mL) and dichloromethane (100 mL) was added 1-chloroethyl chloroformate (5.40 mL) at -78°C and the mixture was stirred at room temperature for 3 days. After concentration under reduced pressure, ethyl acetate (200 mL) was added to the reaction solution. The mixture was washed with saturated brine (100 mL), dried over anhydrous magnesium sulfate and concentrated under reduced pressure to give 1-chloroethyl isopropyl carbonate (7.87 g) as a yellow orange oil.
¹H-NMR (CDCl₃) : 1.33(3H,d,J=6.3Hz), 1.34(3H,d,J=6.3Hz), 1.83(3H,d,J=5.8Hz), 4.88-5.02(1H,m), 6.43(1H,q,J=5.8Hz).

A mixture of 1-chloroethyl isopropyl carbonate (5.00 g), sodium iodide (6.30 g), zinc chloride (205 mg) and carbon disulfide (25 mL) was stirred at room temperature for 4 hrs. Ice water was added to the reaction solution and the mixture was extracted with ether (50 mLx2). The organic layer was washed with aqueous sodium hydrogen carbonate (30 mL) and 10% aqueous sodium thiosulfate (30 mL), dried over anhydrous magnesium sulfate and concentrated under reduced pressure to give 1-iodoethyl isopropyl carbonate (6.18 g) as a black-yellow oil.
¹H-NMR (CDCl₃) : 1.33(3H,d,J=6.2Hz), 1.35(3H,d,J=6.2Hz), 2.24(3H,d,J=6.0Hz), 4.85-5.05(1H,m), 6.77(1H,q,J=6.0Hz).

A mixture of (R)-2-[[[3-methyl-4-(2,2,2-trifluoroethoxy)-2-pyridyl]methyl]sulfinyl]benzimidazole (5.54 g), 1-iodoethyl isopropyl carbonate (5.68 g), cesium carbonate (9.77 g) and acetone (100 mL) was stirred at room temperature for 1 hr. After concentration under reduced pressure, ethyl acetate (200 mL) was added to the reaction solution, and the mixture was washed with water (100 mL), 10% aqueous sodium thiosulfate (100 mL) and saturated brine (100 mL) and dried over anhydrous magnesium sulfate. After concentration under reduced pressure, the residue was purified by silica gel column chromatography (eluted with ethyl acetate:hexane=1:1, then successively with ethyl acetate). Diisopropyl ether was added to allow for solidification and the solid was collected by filtration and washed. It was recrystallized from ethyl acetate and diisopropyl ether to give a less polar diastereomer (1.40 g) of the title compound as a colorless solid.
¹H-NMR (CDCl₃) : 1.15(3H,d,J=6.2Hz), 1.28(3H,d,J=6.2Hz), 2.00(3H,d,J=6.2Hz), 2.37(3H,s), 4.40(2H,q,J=7.8Hz), 4.68-4.88(1H,m), 5.00(1H,d,J=13.7Hz), 5.12(1H,d,J=13.7Hz), 6.66(1H,d,J=5.7Hz), 7.28-7.46(3H,m), 7.70-7.93(2H,m), 8.38(1H,d,J=5.7Hz).

### Example 11

### Isopropyl 1-[2-[[[3-methyl-4-(2,2,2-trifluoroethoxy)-2-pyridinyl]methyl]sulfinyl]-1H-benzimidazol-1-yl]ethyl carbonate

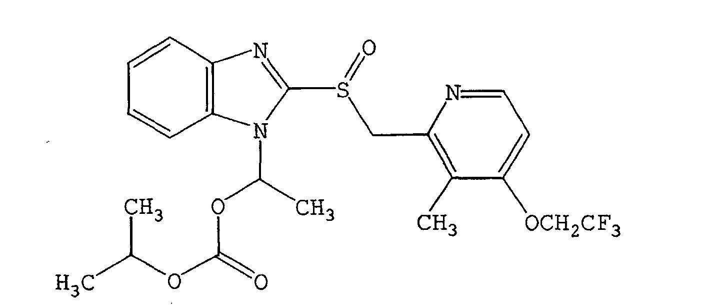

To a solution (30 mL) of 1-chloroethyl isopropyl carbonate (5.15 g) in carbon disulfide were added sodium iodide (9.27 g) and zinc chloride (0.30 g) and the mixture was stirred at room temperature for 2.5 hrs. The reaction solution was poured into ice water (150 mL) and the mixture was extracted 3 times with diethyl ether (50 mL). The organic layers were combined, and washed with 10% aqueous sodium sulfite solution (30 mL), saturated aqueous sodium hydrogen carbonate (30 mL) and water (30 mL). The mixture was dried over anhydrous magnesium sulfate and concentrated under reduced pressure to give 1-iodoethyl isopropyl carbonate (5.01 g) as a liquid.

To a solution (70 mL) of 2-[[[3-methyl-4-(2,2,2-trifluoroethoxy)-2-pyridinyl]methyl]sulfinyl]benzimidazole (2.64 g) and 1-iodoethyl isopropyl carbonate (2.03 g) in acetone was added cesium carbonate (2.56 g) and the mixture was stirred at room temperature for 2 hrs. Acetone was evaporated under reduced pressure and the residue was extracted with ethyl acetate (140 mL) and water (70 mL). The organic layer was separated, and washed with 10% aqueous sodium sulfite solution (30 mL) and saturated brine (30 mL). The mixture was dried over anhydrous magnesium sulfate and concentrated under reduced pressure. The residue was purified by silica gel chromatography (eluted with ethyl acetate:hexane=2:1) and further purified by preparative HPLC (ODS-A manufactured by YMC Co. Ltd., diameter 30 mm, length 250 mm: eluted with acetonitrile:water=33:67, flow rate 20 mL/min) to give a less polar diastereomer (0.296 g) of the title compound as a colorless solid.
¹H-NMR (CDCl₃) : 1.15(3H,d,J=6.0Hz), 1.28(3H,d,J=6.3Hz), 2.00(3H,d,J=6.3Hz), 2.38(3H,s), 4.40(2H,q,J=7.9Hz), 4.78(1H,m), 5.00(1H,d,J=13.8Hz), 5.12(1H,d,J=13.8Hz), 6.67(1H,d,J=5.6Hz), 7.31-7.44(3H,m)7.76(1H,m), 7.88(1H,m), 8.39(1H,d,J=5.6Hz).

### Example 12

### Isopropyl 1-[(S)-2-[[[3-methyl-4-(2,2,2-trifluoroethoxy)-2-pyridinyl]methyl]sulfinyl]-1H-benzimidazol-1-yl]ethyl carbonate

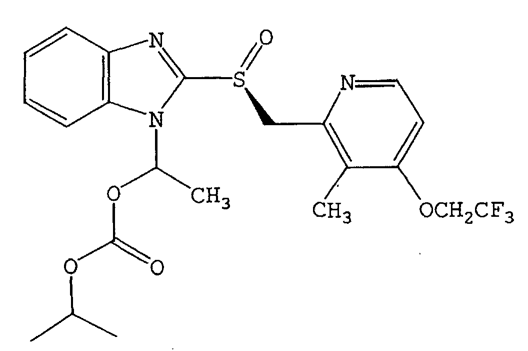

To a solution (100 mL) of (S)-2-[[[3-methyl-4-(2,2,2-trifluoroethoxy)-2-pyridinyl]methyl]sulfinyl]-1H-benzimidazole (3.70 g) and isopropyl 1-iodoethyl carbonate (2.86 g) in acetone was added cesium carbonate (3.62 g) under ice-cooling, and the mixture was stirred for 1.5 hrs. The reaction solution was concentrated under reduced pressure, and the residue was extracted with ethyl acetate and water. The organic layer was separated, and washed with saturated brine, dried over anhydrous magnesium sulfate and concentrated under reduced pressure. The residue was purified by silica gel chromatography (eluted with ethyl acetate:hexane=2:1), and the obtained solid was recrystallized from ethyl acetate-isopropyl ether to give a less polar diastereomer (0.75 g) of the title compound as a colorless solid.
¹H-NMR(CDCl₃): 1.15(3H,d,J=6.2Hz), 1.27(3H,d,J=6.2Hz), 2.00(3H,d,J=6.6Hz), 2.37(3H,s), 4.40(2H,q,J=8.0Hz), 4.80-4.95(1H,m), 5.00(1H,d,J=14.0Hz), 5.12(1H,d,J=14.0Hz), 6.67(1H,d,J=6.0Hz), 7.30-7.50(3H,m), 7.70-7.95(2H,m), 8.38 (1H,d,J=6. 0Hz).

### Example 13

### 1,3-Diethoxy-2-propyl 1-[2-[[[3-methyl-4-(2,2,2-trifluoroethoxy)-2-pyridinyl]methyl]sulfinyl]-1H-benzimidazol-1-yl]ethyl carbonate

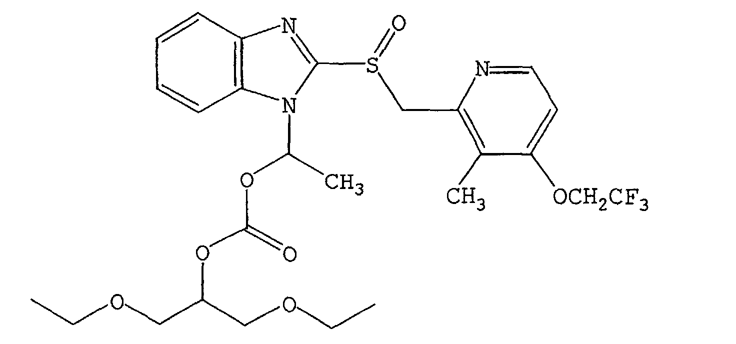

To a solution (30 mL) of 2-[[[3-methyl-4-(2,2,2-trifluoroethoxy)-2-pyridinyl]methyl]thio]-1H-benzimidazole (1.77 g) in acetone were added the compound (3.00 g) of Reference Example 7 and cesium carbonate (2.61 g), and the mixture was stirred at room temperature for 18 hrs. The reaction solution was extracted with ethyl acetate - water, and the organic layer was washed with saturated brine, dried over anhydrous magnesium sulfate and concentrated under reduced pressure. The residue was purified by silica gel chromatography (eluted with ethyl acetate:hexane=2:1) to give 1,3-diethoxy-2-propyl 1-[2-[[[3-methyl-4-(2,2,2-trifluoroethoxy)-2-pyridinyl]methyl]thio]-1H-benzimidazol-1-yl]ethyl carbonate (0.45 g) as a colorless solid.
¹H-NMR(CDCl₃): 0.97(3H,t,J=7.0Hz), 1.17(3H,t,J=7.0Hz), 1.87(3H,d,J=6.2Hz), 2.35(3H,s), 3.20-3.70(8H,m), 4.40(2H,q,J=5.8Hz), 4.70-4.95(1H,m), 4.85(2H,s), 6.66(1H,d,J=6.0Hz), 6.87(1H,q,J=6.2Hz), 7.10-7.30(2H,m), 7.55-7.75(2H,m), 8.36(1H,d,J=6.0Hz).

To a solution (10 mL) of 1,3-diethoxy-2-propyl 1-[2-[[[3-methyl-4-(2,2,2-trifluoroethoxy)-2-pyridinyl]methyl]thio]-1H-benzimidazol-1-yl]ethyl carbonate (0.44 g) in toluene was added 3-chloroperoxybenzoic acid (content: ca. 65%: 0.15 g) under ice-cooling, and the mixture was stirred for 3 hrs. The reaction solution was extracted with ethyl acetate - water, and the organic layer was washed with 10% aqueous sodium thiosulfate solution and saturated brine, dried over anhydrous magnesium sulfate and concentrated under reduced pressure. The residue was purified by silica gel chromatography (eluted with ethyl acetate) and crystallized from diisopropyl ether to give a less polar diastereomer (0.25 g) of the title compound as a colorless solid.
¹H-NMR (CDCl₃) : 0.87(3H,t,J=7.0Hz), 1.17(3H,t,J=7.0Hz), 2.01(3H,d,J=6.6Hz), 2.37(3H,s), 3.10-3.65(8H,m), 4.40(2H,q,J=8.2Hz), 4.80-4.95(1H,m), 5.00(1H,d,J=14.0Hz), 5.12(1H,d,J=14.0Hz), 6.67(1H,d,J=5.8Hz), 7.30-7.50(3H,m), 7.70-7.90(2H,m), 8.38(1H,d,J=5.8Hz).

### Example 14

### 1,3-Dimethoxy-2-propyl 1-[2-[[[3-methyl-4-(2,2,2-trifluoroethoxy)-2-pyridinyl]methyl]sulfinyl]-1H-benzimidazol-1-yl]ethyl carbonate

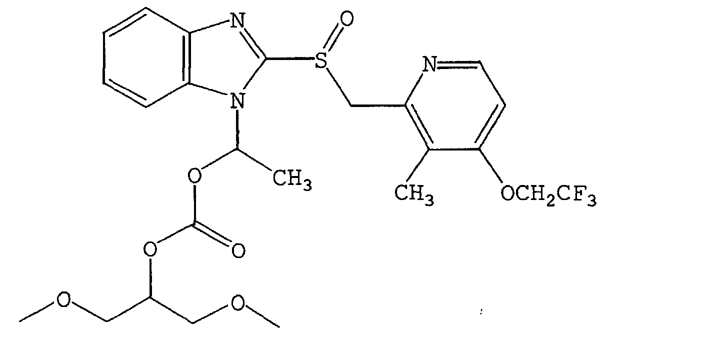

To a solution (20 mL) of 2-[[[3-methyl-4-(2,2,2-trifluoroethoxy)-2-pyridinyl]methyl]thio]-1H-benzimidazole (2.64 g) in acetone were added the compound (3.40 g) of Reference Example 9 and cesium carbonate (3.49 g) and the mixture was stirred at room temperature for 18 hrs. The reaction solution was extracted with ethyl acetate - water, and the organic layer was washed with saturated brine, dried over anhydrous magnesium sulfate and concentrated under reduced pressure. The residue was purified by silica gel chromatography (eluted with ethyl acetate:hexane=2:1) to give 1,3-dimethoxy-2-propyl 1-[2-[[[3-methyl-4-(2,2,2-trifluoroethoxy)-2-pyridinyl]methyl]thio]-1H-benzimidazol-1-yl]ethyl carbonate (1.48 g) as a colorless solid.
¹H-NMR(CDCl₃): 1.87(3H,d,J=6.2Hz), 2.35(3H,s), 3.17(3H,s), 3.34(3H,s), 3.42(2H,d,J=5.2Hz), 3.55(2H,d,J=5.2Hz), 4.40(2H,q,J=8.0Hz), 4.80-5.00(3H,m), 6.65(1H,d,J=5.8Hz), 6.67(1H,q,J=6.2Hz), 7.15-7.35(2H,m), 7.55-7.75(2H,m), 8.36(1H,d,J=5.8Hz) .

To a solution (20 mL) of 1,3-dimethoxy-2-propyl 1-[2-[[[3-methyl-4-(2,2,2-trifluoroethoxy)-2-pyridinyl]methyl]thio]-1H-benzimidazol-1-yl]ethyl carbonate (1.45 g) in toluene was added 3-chloroperoxybenzoic acid (content: ca. 65%: 0.506 g) under ice-cooling, and the mixture was stirred for 3 hrs. The reaction solution was extracted with ethyl acetate - water, and the organic layer was washed with 10% aqueous sodium thiosulfate solution and saturated brine, dried over anhydrous magnesium sulfate and concentrated under reduced pressure. The residue was purified by silica gel chromatography (eluted with ethyl acetate) and recrystallized from ethyl acetate - diisopropyl ether to give a less polar diastereomer (0.85 g) of the title compound as a colorless solid.
¹H-NMR(CDCl₃): 2.01(3H,d,J=6.6Hz), 2.36(3H,s), 3.10(3H,s), 3.35(3H,s), 3.38(2H,d,J=5.0Hz), 3.53(2H,d,J=5.0Hz), 4.40(2H,q,J=7.6Hz), 4.80-4.95(1H,m), 5.00(1H,d,J=13.8Hz), 5.11(1H,d,J=13.8Hz), 6.66(1H,d,J=6.0Hz), 7.30-7.50(3H,m), 7.70-7.90(2H,m), 8.37(1H,d,J=6.0Hz).

### Example 15

### 1-[2-[[[3-Methyl-4-(2,2,2-trifluoroethoxy)-2-pyridinyl]methyll]sulfinyl]-1H-benzimidazol-1-yl] ethyl tetrahydro-2H-pyran-4-yl carbonate

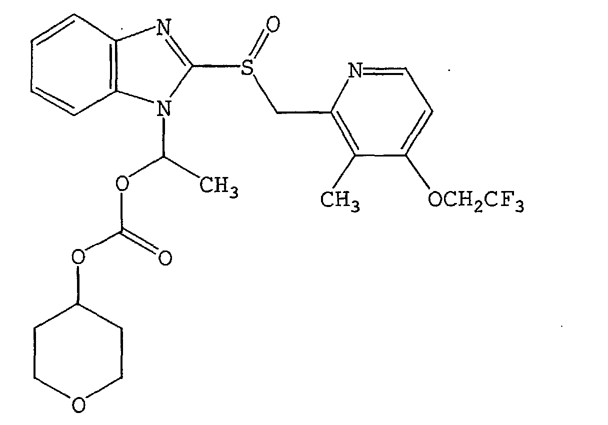

To a solution (20 mL) of 2-[[[3-methyl-4-(2,2,2-trifluoroethoxy)-2-pyridinyl]methyl]thio]-1H-benzimidazole (2.47 g) in acetone were added the compound (3.00 g) of Reference Example 11 and cesium carbonate (3.25 g) and the mixture was stirred at room temperature for 3 hrs. The reaction solution was extracted with ethyl acetate - water, and the organic layer was washed with saturated brine, dried over anhydrous magnesium sulfate and concentrated under reduced pressure. The residue was purified by silica gel chromatography (eluted with ethyl acetate:hexane=2:1) to give 1-[2-[[[3-methyl-4-(2,2,2-trifluoroethoxy)-2-pyridinyl]rnethyl]thio]-1H-benzimidazol-1-yl]ethyl tetrahydro-2H-pyran-4-yl carbonate (0.85 g) as a colorless solid.
¹H-NMR(CDCl₃): 1.50-2.10(4H,m), 1.88(3H,d,J=6.4Hz), 2.36(3H,s), 3.35-3.55(2H,m), 3.80-4.00(2H,m), 4.40(2H,q,J=7.8Hz), 4.65-4.85(1H,m), 4.86(2H,s), 6.66(1H,d,J=5.8Hz), 6.88(1H,q,J=6.4Hz), 7.20-7.30(2H,m), 7.55-7.75 (2H,m) , 8.36 (1H,d,J=5.8Hz).

To a solution (15 mL) of 2-[2-[[[3-methyl-4-(2,2,2-trifluoroethoxy)-2-pyridinyl]methyl]thio]-1H-benzimidazol-1-yl]ethyl tetrahydro-2H-pyran-4-yl carbonate (0.70 g) in toluene was added 3-chloroperoxybenzoic acid (content: ca. 65%: 0.252 g) under ice-cooling, and the mixture was stirred for 3 hrs. The reaction solution was extracted with ethyl acetate - water, and the organic layer was washed with 10% aqueous sodium thiosulfate solution and saturated brine, dried over anhydrous magnesium sulfate and concentrated under reduced pressure. The residue was purified by silica gel chromatography (eluted with ethyl acetate), and recrystallized from ethyl acetate - diisopropyl ether to give a less polar diastereomer (0.48 g) of the title compound as a colorless solid.
¹H-NMR (CDCl₃) : 1.50-1.90(4H,m), 2.02(3H,d,J=6.2Hz), 2.38(3H,s), 3.30-3.55(2H,m), 3.75-4.00(2H,m), 4.41(2H,q,J=7.6Hz), 4.60-4.80(1H,m), 5.00(1H,d,J=13.8Hz), 5.12(1H,d,J=13.8Hz), 6.67(1H,d,J=5.8Hz), 7.30-7.50(3H,m), 7.70-7.95(2H,m), 8.37(1H,d,J=5.8Hz).

### Example 16

### 2-Methoxyethyl 1-[2-[[[3-methyl-4-(2,2,2-trifluoroethoxy)-2-pyridinyl]methyl]sulfinyl]-1H-benzimidazol-1-yl]ethyl carbonate

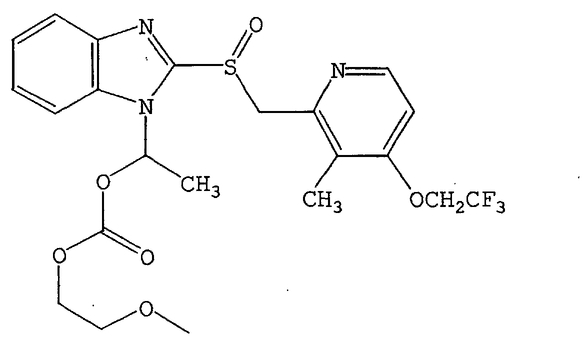

To a solution (20 mL) of 2-[[[3-methyl-4-(2,2,2-trifluoroethoxy)-2-pyridinyl-2-methyl]thio]-1H-benzimidazole (3.29 g) in acetone were added compound (3.65 g) of Reference Example 13 and sodium hydrogen carbonate (5.59 g) and the mixture was stirred at room temperature for 18 hrs. The reaction solution was extracted with ethyl acetate - water and the organic layer was washed with saturated brine, dried over anhydrous magnesium sulfate and concentrated under reduced pressure. The residue was purified by silica gel chromatography (eluted with ethyl acetate:hexane=2:1) to give 2-methoxyethyl 1-[2-[[[3-methyl-4-(2,2,2-trifluoroethoxy)-2-pyridinyl]methyl]thio]-1H-benzimidazol-1-yl] ethyl carbonate (1.99 g) as a colorless solid.
¹H-NMR (CDCl₃) : 1.87(3H,d,J=6.2Hz), 2.35(3H,s), 3.32(3H,s), 3.55(2H,t,J=4.4Hz), 4.10-4.50(4H,m), 4.85(2H,s), 6.66(1H,d,J=5.8Hz), 6.89(1H,q,J=6.2Hz), 7.15-7.30(2H,m), 7.50-7.75(2H,m), 8.36(1H,d,J=5.8Hz).

To a solution (25 mL) of 2-methoxyethyl 1-[2-[[[3-methyl-4-(2,2,2-trifluoroethoxy)-2-pyridinyl]methyl]thio]-1H-benzimidazol-1-yl]ethyl carbonate (1.50 g) in toluene was added 3-chloroperoxybenzoic acid (content: ca. 65%: 0.569 g) under ice-cooling, and the mixture was stirred for 1 hr. The reaction solution was extracted with ethyl acetate - water, and the organic layer was washed with 10% aqueous sodium thiosulfate solution and saturated brine, dried over anhydrous magnesium sulfate and concentrated under reduced pressure. The residue was purified by silica gel chromatography (eluted with ethyl acetate) and recrystallized from ethyl acetate-diisopropyl ether to give a less polar diastereomer (0.90 g) of the title compound as a colorless solid.
¹H-NMR(CDCl₃): 2.01(3H,d,J=6.2Hz), 2.37(3H,s), 3.31(3H,s), 3.53(2H,t,J=4.8Hz), 4.05-4.50(4H,m), 4.99(1H,d,J=14.0Hz), 5.12(1H,d,J=14.0Hz), 6.67(1H,d,J=5.8Hz), 7.30-7.50(3H,m), 7.70-7.90(2H,m), 8.38(1H,d,J=5.8Hz).

### Example 17

### 1-[2-[[[3-Methyl-4-(2,2,2-trifluoroethoxy)-2-pyridinyl]methyl]sulfinyl]-1H-benzimidazol-1-yl] ethyl 2,2-bis(methoxymethyl)propanoate

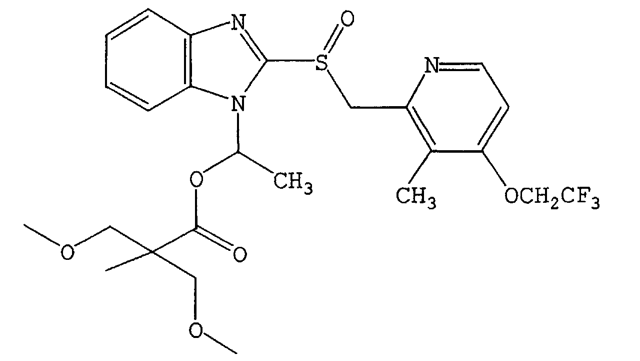

To a solution (10 mL) of 2-[[[3-methyl-4-(2,2,2-trifluoroethoxy)-2-pyridinyl]methyl]thio]-1H-benzimidazole (0.67 g) in acetone were added the compound (1.20 g) of Reference Example 15 and cesium carbonate (0.912 g) and the mixture was stirred at room temperature for 18 hrs. The reaction solution was extracted with ethyl acetate - water, and the organic layer was washed with saturated brine, dried over anhydrous magnesium sulfate and concentrated under reduced pressure. The residue was purified by silica gel chromatography (eluted with ethyl acetate:hexane=2:1) to give 1-[2-[[[3-methyl-4-(2,2,2-trifluoroethoxy)-2-pyridinyl]methyl]thio]-1H-benzimidazol-1-yl]ethyl 3-methoxy-2-(methoxymethyl)-2-methylpropanoate (1-[2-[[[3-methyl-4-(2,2,2-trifluoroethoxy)-2-pyridinyl]methyl]thio]-1H-benzimidazol-1-yl]ethyl 2,2-bis(methoxymethyl)propanoate) (0.280 g) as a colorless solid.
¹H-NMR(CDCl₃): 1.15(3H,s), 1.83(3H,d,J=6.2Hz), 2.35(3H,s), 3.13(3H,s), 3.19(3H,s), 3.41(2H,s), 3.44(2H,s), 4.40(2H,q,J=8.2Hz), 4.85(2H,s), 6.66(1H,d,J=5.8Hz), 7.03(1H,q,J=6.2Hz), 7.10-7.30(2H,m), 7.50-7.60(1H,m), 7.62-7.75(1H,m), 8.36(1H,d,J=5.8Hz).

To a solution (10 mL) of 1-[2-[[[3-methyl-4-(2,2,2-trifluoroethoxy)-2-pyridinyl]methyl]thio]-1H-benzimidazol-1-yl]ethyl 2,2-bis(methoxymethyl)propanoate (0.27 g) in toluene was added 3-chloroperoxybenzoic acid (content: ca. 65%: 95 mg) under ice-cooling, and the mixture was stirred for 1 hr. The reaction solution was extracted with ethyl acetate - water, and the organic layer was washed with 10% aqueous sodium thiosulfate solution and saturated brine, dried over anhydrous magnesium sulfate and concentrated under reduced pressure. The residue was purified by silica gel chromatography (eluted with ethyl acetate:hexane=2:1) and recrystallized from ethyl acetate - diisopropyl ether to give a less polar diastereomer (0.12 g) of the title compound as a colorless solid.
¹H-NMR (CDCl₃) : 1.14(3H,s), 1.98(3H,d,J=6.2Hz), 2.37(3H,s), 3.05(3H,s), 3.12(3H,s), 3.30-3.50(4H,m), 4.40(2H,q,J=8.0Hz), 5.00(1H,d,J=13.8Hz), 5.12(1H,d,J=13.8Hz), 6.66(1H,d,J=5.6Hz), 7.25-7.50(3H,m), 7.65-7.75(1H,m), 7.80-7.90(1H,m), 8.38 (1H,d, J=5. 6Hz).

### Example 18

### 1-[2-[[[3-Methyl-4-(2,2,2-trifluoroethoxy)-2-pyridinyl]methyl]sulfinyl]-1H-benzimidazol-1-yl]ethyl cyclopentanecarboxylate

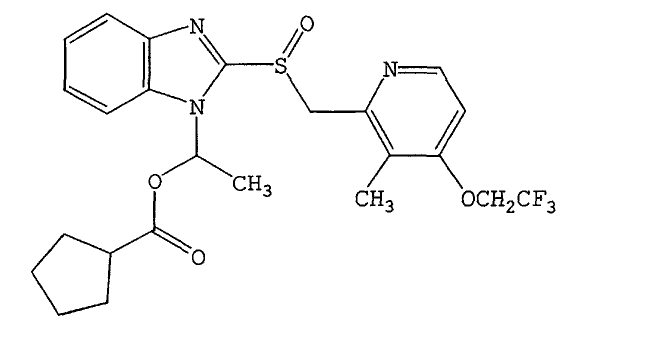

Using the compound of Reference Example 16 and by a similar operation as in Example 17, a less polar diastereomer (1.80 g) of the title compound was synthesized.
¹H-NMR(CDCl₃): 1.40-2.00(8H,m), 1.96(3H,d,J=6.2Hz), 2.37(3H,s), 2.60-2.90(1H,m), 4.40(2H,q,J=7.6Hz), 5.00(1H,d,J=13.8Hz), 5.12(1H,d,J=13.8Hz), 6.66(1H,d,J=5.8Hz), 7.30-7.45(3H,m), 7.65-7.75(1H,m), 7.80-7.90(1H,m), 8.38(1H,d,J=5.8Hz).

### Example 19

### Cyclopentyl 1-[2-[[[3-methyl-4-(2,2,2-trifluoroethoxy)-2-pyridinyl]methyl]sulfinyl]-1H-benzimidazol-1-yl] ethyl carbonate

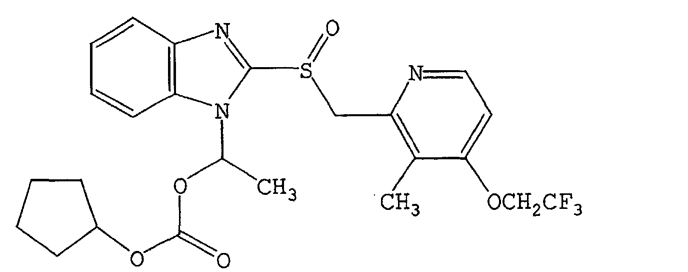

A mixture of 2-[[[3-methyl-4-(2,2,2-trifluoroethoxy)-2-pyridinyl]methyl]thio]-1H-benzimidazole (3.18 g), cyclopentyl 1-iodoethyl carbonate (3.84 g), sodium hydrogen carbonate (3.78 g) and acetonitrile (20 mL) was stirred at room temperature for 24 hrs. Water (50 mL) was added to the reaction solution and the mixture was extracted with ethyl acetate (50 mL). The ethyl acetate layer was washed with saturated brine (50 mL) and dried over anhydrous sodium sulfate. After filtration, the filtrate was concentrated under reduced pressure and the obtained residue was purified by silica gel flash column chromatography (eluted with ethyl acetate:hexane=4:1) to give cyclopentyl 1-[2-[[[3-methyl-4-(2,2,2-trifluoroethoxy)-2-pyridinyl]methyl]thio]-1H-benzimidazol-1-yl]ethyl carbonate (2.69 g) as a pale-yellow orange powder.
¹H-NMR(CDCl₃) : 1.52-2.00(8H,m), 1.86(3H,d,J=6.2Hz), 2.35(3H,s), 4.40(2H,q,J=8.0Hz), 4.86(2H,s), 4.98-5.03(1H,m), 6.66(1H,d,J=6.0Hz), 6.89(1H,q,J=6.2Hz), 7.17-7.27(2H,m), 7.56-7.72(2H,m), 8.36(1H,d,J=6.0Hz).

To a solution of 1-[2-[[[3-methyl-4-(2,2,2-trifluoroethoxy)-2-pyridinyl]methyl]thio]-1H-benzimidazol-1-yl]ethyl carbonate (2.62 g) in toluene (10 mL) was added 3-chloroperoxybenzoic acid (content: ca. 65%: 1.27 g) at 0°C. The mixture was stirred at 0°C for 3 hrs. Water (50 mL) was added to the reaction solution and the mixture was extracted with ethyl acetate (50 mL). The ethyl acetate layer was washed with aqueous sodium hydrogen carbonate (50 mL) and saturated brine (50 mL) and dried over anhydrous sodium sulfate. After concentration under reduced pressure, the residue was purified by silica gel flash column chromatography (eluted with ethyl acetate:hexane=5:1-10:1) and crystallized from ethyl acetate-diisopropyl ether to give a less polar diastereomer (707 mg) of the title compound as white powder crystals.
¹H-NMR (CDCl₃) : 1.40-1.90(8H,m) , 2.00(3H,d,J=6.6Hz) , 2.37(3H,s), 4.44(2H,q,J-8.0Hz), 4.96-5.03(1H,m), 5.00(1H,d,J=13.6Hz), 5.12(1H,d,J=13.6Hz), 6.67(1H,d,J=6.0Hz), 7.26-7.44(3H,m), 7.71-7.76(1H,m), 7.83-7.89(1H,m), 8.38(1H,d,J=6.0Hz).

### Example 20

### 2-(Acetylamino)ethyl 1-[2-[[[3-methyl-4-(2,2,2-trifluoroethoxy)-2-pyridinyl]methyl]sulfinyl]-1H-benzimidazol-1-yl]ethyl carbonate

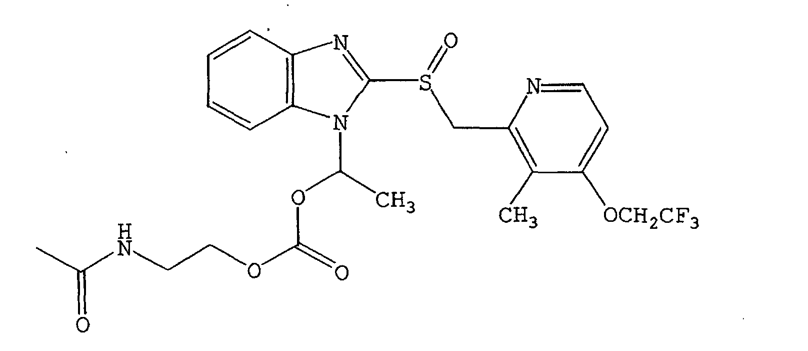

A mixture of 2-[[[3-methyl-4-(2,2,2-trifluoroethoxy)-2-pyridinyl]methyl]thio]-1H-benzimidazole (626 mg), 2-(acetylamino)ethyl 1-iodoethyl carbonate (800 mg), sodium hydrogen carbonate (743 mg) and acetonitrile (20 mL) was stirred at room temperature for 20 hrs. Water (50 mL) was added to the reaction solution and the mixture was extracted with ethyl acetate (100 mL). The ethyl acetate layer was washed with saturated brine (100 mL) and dried over anhydrous sodium sulfate. After filtration, the filtrate was concentrated under reduced pressure and the obtained residue was purified by silica gel flash column chromatography (eluted with ethyl acetate:hexane=1:1) and crystallized from diisopropyl ether to give 2-(acetylamino)ethyl 1-[2-[[[3-methyl-4-(2,2,2-trifluoroethoxy)-2-pyridinyl]methyl]thio]-1H-benzimidazol-1-yl]ethyl carbonate (265 mg) as pale-yellow powder crystals.
¹H-NMR (CDCl₃) : 1.87(3H,s), 1.90(3H,d,J=6.2Hz), 2.36(3H,s), 3.46(2H,q,J=6.0Hz), 4.18(2H,t,J=6.0Hz), 4.45(2H,q,J=8.2Hz), 4.82(2H,d,J=13.6Hz), 4.89(2H,d,J=13.6Hz), 5.75(1H,bs), 6.66(1H,d,J=6.2Hz), 6.84(1H,q,J=6.2Hz), 7.20-7.31(2H,m), 7.56-7.60(1H,m), 7.68-7.72(1H,m), 8.35(1H,d,J=6.2Hz).

To a solution of 2-(acetylamino)ethyl 1-[2-[[[3-methyl-4-(2,2,2-trifluoroethoxy)-2-pyridinyl]methyl]thio]-1H-benzimidazol-1-yl]ethyl carbonate (500 mg) in toluene (5 mL) was added 3-chloroperoxybenzoic acid (content: ca. 65%: 277 mg) at 0°C. The mixture was stirred at room temperature for 2 hrs. and water (50 mL) was added to the reaction solution. The solution was extracted with ethyl acetate (50 mLx2). The ethyl acetate layer was washed with aqueous sodium hydrogen carbonate (100 mL) and saturated brine (100 mL), dried over anhydrous sodium sulfate and concentrated under reduced pressure. The obtained residue was purified by silica gel flash column chromatography (eluted with acetone:hexane=1:1-2:1), and crystallized from diisopropyl ether to give a less polar diastereomer (319 mg) of the title compound as white powder crystals.
¹H-NMR(CDCl₃): 1.74(3H,s), 2.03(3H,d,J=6.2Hz), 2.37(3H,s), 3.36-3.48(2H,m), 4.02-4.14(1H,m), 4.25-4.34(1H,m), 4.39(2H,q,J=6.6Hz), 5.03(1H,d,J=6.4Hz), 5.26(1H,d,J=6.4Hz), 6.48(1H,bs), 6.63(1H,d,J=5.4Hz), 7.20(1H,q,J=6.2Hz), 7.33-7.48(2H,m), 7.64-7.69(1H,m), 7.81-7.86(1H,m), 8.23(1H,d, J=5.4Hz).

### Example 21

### Diethyl 3-[[[1-[2-[[[3-methyl-4-(2,2,2-trifluoroethoxy)-2-pyridinyl]methyl]sulfinyl]-1H-benzimidazol-1-yl]ethoxy]carbonyl]oxy]-1,5-pentanedioate

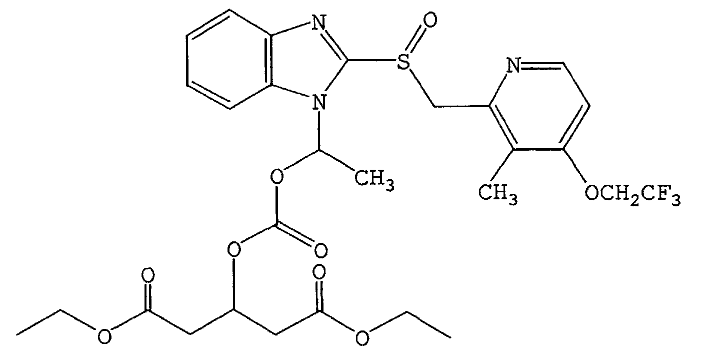

A mixture of 2-[[[3-methyl-4-(2,2,2-trifluoroethoxy)-2-pyridinyl]methyl]thio]-1H-benzimidazole (1.91 g), diethyl 3-[[(1-iodoethoxy)carbonyl]oxy]-1,5-pentanedioate (3.26 g), sodium hydrogen carbonate (2.27 g) and acetonitrile (50 mL) was stirred at room temperature for 20 hrs. Water (100 mL) was added to the reaction solution and the mixture was extracted with ethyl acetate (100 mL). The ethyl acetate layer was washed with saturated brine (100 mL) and dried over anhydrous sodium sulfate. After filtration, the filtrate was concentrated under reduced pressure and the obtained residue was purified by column chromatography [Chromatorex NH silica gel (FUJI SILYSIA CHEMICAL LTD.), eluted with ethyl acetate:hexane=1:1] and crystallized from diisopropyl ether to give diethyl 3-[[[1-[2-[[[3-methyl-4-(2,2,2-trifluoroethoxy)-2-pyridinyl]methyl]thio]-1H-benzimidazol-1-yl)ethoxy]carbonyl]oxy]-1,5-pentanedioate (1.27 g) as pale-yellow powder crystals.
¹H-NMR (CDCl₃) : 0.99(3H,t,J=7.0Hz), 1.25 (3H,t,J=7.2Hz), 2.86(3H,d,J=6.4Hz), 2.35(3H,s), 2.64(2H,d,J=6.6Hz), 2.72(2H,d,J=6.6Hz), 3.74-3.96(2H,m), 4.13(2H,q,J=7.0Hz), 4.37(2H,q,J=7.2Hz), 4.82(1H,d,J=13.8Hz), 4.88(1H,d,J=13.8Hz), 5.32(1H,d,J=6.6Hz), 6.66(1H,d,J=5.8Hz), 6.87(1H,q.J=6.4Hz), 7.16-7.29(2H,m), 7.52-7.58(1H,m), 7.66-7.70(1H,m), 8.36(1H,d,J=5.2Hz).

To a solution of diethyl 3-[[[1-[2-[[[3-methyl-4-(2,2,2-trifluoroethoxy)-2-pyridinyl]methyl]thio]-1H-benzimidazol-1-yl)ethoxy]carbonyl]oxy]-1,5-pentadioate (500 mg) in toluene (5 mL) was added 3-chloroperoxybenzoic acid (content: ca. 65%: 233 mg) at 0°C. The mixture was stirred at room temperature for 3 hrs., and water (50 mL) was added to the reaction solution. The mixture was extracted with ethyl acetate (100 mL). The ethyl acetate layer was washed with aqueous sodium hydrogen carbonate (100 mL) and saturated brine (100 mL) and dried over anhydrous sodium sulfate. It was concentrated under reduced pressure and the obtained residue was purified by silica gel flash column chromatography (eluted with ethyl acetate:hexane=1:4) and crystallized from diisopropyl ether to give a less polar diastereomer (302 mg) of the title compound as white powder crystals.
¹H-NMR (CDCl₃) : 0.94(3H,t,J=7.0Hz), 1.25(3H,t,J=7.0Hz), 2.00(3H,d,J=6.2Hz), 2.36(3H,s), 2.60(2H,d,J=6.2Hz), 2.72(2H,d,J=6.2Hz), 3.65-3.88(2H,m), 4.14(2H,q,J=7.0Hz), 4.41(2H,q.J=7.0Hz), 5.03(1H,d,J=14.0Hz), 5.11(1H,d,J=14.0Hz), 5.31(1H,q,J=6.2Hz), 6.67(1H,d,J=6.0Hz), 7.31-7.42(3H,m), 7.67-7.76(1H,m), 7.82-7.88(1H,m), 8.37(1H,d,J=6.0Hz).

### Example 22

### 1-[2-[[[3-Methyl-4-(2,2,2-trifluoroethoxy)-2-pyridinyl]methyl]sulfinyl]-1H-benzimidazol-1-yl]ethyl acetate

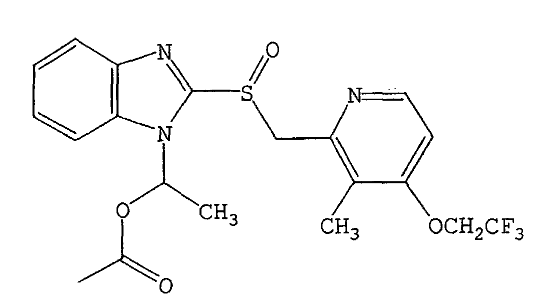

A mixture of 2-[[[3-methyl-4-(2,2,2-trifluoroethoxy)-2-pyridinyl]methyl]thio]-1H-benzimidazole (3.41 g), 1-iodoethyl acetate(3.1 g), sodium hydrogen carbonate (2.44 g), cesium chloride (3.25 g) and acetonitrile (20 mL) was stirred at room temperature for 20 hrs. Water (200 mL) was added to the reaction solution, and the mixture was extracted with ethyl acetate (100 mL). The ethyl acetate layer was washed with water (100 mL) and saturated brine (100 mL), dried over anhydrous sodium sulfate and concentrated under reduced pressure. The obtained residue was purified by silica gel flash column chromatography (eluted with acetone:hexane=1:10-1:4) and crystallized from diisopropyl ether to give 1-[2-[[[3-methyl-4-(2,2,2-trifluoroethoxy)-2-pyridinyl]methyl]thio]-1H-benzimidazol-2-yl]ethyl acetate (975 mg) as pale-yellow powder crystals.
¹H-NMR (CDCl₃) : 1.83(3H,d,J=6.2Hz), 2.05(3H,s), 2.35(3H,s), 4.40(2H,q,J=8.0Hz), 4.85(2H,s), 6.66(1H,d,J=5.4Hz), 7.02(1H,q,J=6.2Hz), 7.18-7.39(2H,m), 7.52-7.59(1H,m), 7.65-7.72(1H,m), 8.37(1H,d,J=5.4Hz).

To a solution of 1-[2-[[[3-methyl-4-(2,2,2-trifluoroethoxy)-2-pyridinyl]methyl]thio]-1H-benzimidazol-1-yl]ethyl acetate (500 mg) in toluene (5 mL) was added 3-chloroperoxybenzoic acid (content: ca. 65%: 332 mg) at 0°C. The mixture was stirred at room temperature for 2 hrs. Water (100 mL) was added to the reaction solution and the mixture was extracted with ethyl acetate (100 mL). The ethyl acetate layer was washed with aqueous sodium hydrogen carbonate (100 mL) and saturated brine (100 mL), dried over anhydrous sodium sulfate and concentrated under reduced pressure. The obtained residue was purified by silica gel flash column chromatography (eluted with ethyl acetate:hexane=4:1-10:1) and crystallized from ethyl acetate - diisopropyl ether to give a less polar diastereomer (300 mg) of the title compound as white powder crystals.
¹H-NMR (CDCl₃) : 1.98(3H,d,J=6.6Hz), 2.07(3H,s), 2.38(3H,s), 4.40(2H,q,J=8.0Hz), 4.98(1H,d,J=14.0Hz), 5.12(1H,d,J=14.0Hz), 6.66(1H,d,J=5.8Hz), 7.33-7.45(3H,m), 7.69-7.73(1H,m), 7.83-7.90(1H,m), 8.37(1H,d,J=5.8Hz).

### Example 23

### 1,3-Dioxan-5-yl 1-[2-[[[3-methyl-4-(2,2,2-trifluoroethoxy)-2-pyridinyl]methyl]sulfinyl]-1H-benzimidazol-1-yl]ethyl carbonate

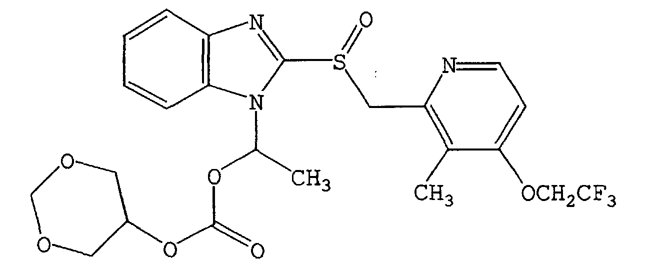

A mixture of 2-[[[3-methyl-4-(2,2,2-trifluoroethoxy)-2-pyridinyl]methyl]thio]-1H-benzimidazole (4.34 g), 1,3-dioxan-5-yl 1-iodoethyl carbonate (5.57 g), sodium hydrogen carbonate (3.1 g), cesium chloride (4.14 g) and acetonitrile (20 mL) was stirred at room temperature for 20 hrs. Water (100 mL) was added to the reaction solution and the mixture was extracted with ethyl acetate (100 mLx2). The ethyl acetate layer was washed with water (100 mL) and saturated brine (100 mL), dried over anhydrous sodium sulfate and concentrated under reduced pressure. The obtained residue was purified by silica gel flash column chromatography (eluted with acetone:hexane=1:5-1:2), and crystallized from diisopropyl ether to give 1,3-dioxan-5-yl 1-[2-[[[3-methyl-4-(2,2,2-trifluoroethoxy)-2-pyridinyl]methyl]thio]-1H-benzimidazol-1-yl] ethyl carbonate (2.71 g) as pale-yellow powder crystals.
¹H-NMR(CDCl₃): 1.89(3H,d,J=6.2Hz), 2.35(3H,s), 3.87-3.90(1H,m), 3.98-4.02(1H,m), 4.40(2H,q,J=8.0Hz), 4.51 (1H, quintet, J=3.0Hz), 4.79(1H,d,J=6.2Hz), 4.85(2H,s), 4.92(1H,d,J=6.2Hz), 6.65(1H,d,J=5.8Hz), 6.89(1H,q,J=6.2Hz), 7.18-7.30(2H,m), 7.57-7.73(2H,m), 8.36(1H,d,J=5.8Hz).

To a solution of 1,3-dioxan-5-yl 1-[2-[[[3-methyl-4-(2,2,2-trifluoroethoxy)-2-pyridinyl]methyl]thio]-1H-benzimidazol-1-yl]ethyl carbonate (1.05 g) in toluene (150 mL)-tetrahydrofuran (15 mL) was added 3-chloroperoxybenzoic acid (content: ca. 65%: 584 mg) at 0°C. The mixture was stirred at room temperature for 2 hrs. and ethyl acetate (100 mL) was added. The mixture was washed with water (100 mL), aqueous sodium hydrogen carbonate (100 mL) and saturated brine (100 mL), dried over anhydrous sodium sulfate and concentrated under reduced pressure. The obtained residue was purified by silica gel flash column chromatography (eluted with ethyl acetate:hexane=1:5-1:1, then successively with ethyl acetate) and crystallized from ethyl acetate - diisopropyl ether to give a less polar diastereomer (828 mg) of the title compound as white powder crystals.
¹H-NMR (CDCl₃) : 2.03(3H,d,J=6.6Hz), 2.37(3H,s), 3.86-3.87(2H,m), 3.97-4.00(2H,m), 4.42(2H,q,J=B.0Hz), 4.49(1H,quintet,J=3.0Hz}, 4.75(1H,d,J=6.2Hz), 4.93(1H,d,J=6.2Hz), 5.00(1H,d,J=13.8Hz), 5.11(1H,d,J=13.8Hz), 6.66(1H,d,J=5.8Hz), 7.33-7.45(3H,m), 7.75-7.82(1H,m), 7.84-7.88(1H,m), 8.36(1H,d,J=5.8Hz).

### Example 24

### 1-[2-[[[3-Methyl-4-(2,2,2-trifluoroethoxy)-2-pyridinyl]methyl]sulfinyl]-1H-benzimidazol-1-yl]ethyl cyclohexanecarboxylate

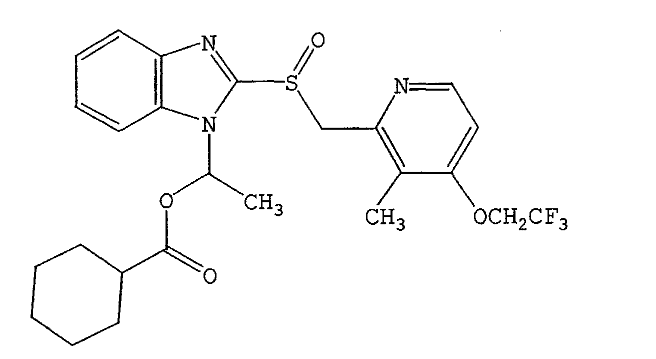

To a mixture of 2-[[[3-methyl-4-(2,2,2-trifluoroethoxy)-2-pyridinyl]methyl]thio]-1H-benzimidazole (3.13 g), cesium carbonate (3.46 g) and acetone (50 mL) was added dropwise 1-iodoethyl cyclohexanecarboxylate (2.50 g). After stirring at room temperature for 4 hrs., insoluble materials were filtered off and the filtrate was concentrated under reduced pressure. The residue was purified by silica gel chromatography (eluted with ethyl acetate:hexane=1 then 2:1) to give 1-[2-[[[3-methyl-4-(2,2,2-trifluoroethoxy)-2-pyridinyl]methyl]thio]-1H-benzimidazol-1-yl]ethyl cyclohexanecarboxylate (1.72 g) as a colorless oil.
¹H-NMR(CDCl₃) : 1.10-2.00(10H,m), 1.81(3H,d,J=6.2Hz), 2.20-2.40(1H,m), 2.35(3H,s), 4.40(2H,q,J=7.9Hz), 4.85(2H,s), 6.65(1H,d,J=5.9Hz), 7.00(1H,q,J=6.2Hz), 7.16-7.30(2H,m), 7.50-7.62(1H,m), 7.64-7.73(1H,m), 8.36(1H,d,J=5.9Hz).

To a solution (30 mL) of 1-[2-[[[3-methyl-4-(2,2,2-trifluoroethoxy)-2-pyridinyl]methyl]thio]-1H-benzimidazol-1-yl]ethyl cyclohexanecarboxylate (1.65 g) in toluene was added 3-chloroperoxybenzoic acid (content: ca. 65%: 0.865 g) by small portions under ice-cooling. After stirring under ice-cooling for 1 hr., ethyl acetate (100 mL) was added and the mixture was washed with aqueous sodium hydrogen carbonate (50 mL), aqueous sodium thiosulfate solution (50 mL) and brine (50 mL). The mixture was dried over anhydrous magnesium sulfate and concentrated under reduced pressure. The residue was purified by silica gel chromatography (eluted with ethyl acetate:hexane=2:2, then successively with ethyl acetate). Crystallization from diisopropyl ether and recrystallization from ethyl acetate - diisopropyl ether gave a less polar diastereomer (1.10 g) of the title compound as a colorless solid.
¹H-NMR(CDCl₃): 1.10-2.00(10H,m), 1.96(3H,d,J=6.6Hz), 2.22-2.42(1H,m), 2.37(3H,s), 4.40(2H,q,J=7.8Hz), 5.01(1H,d,J=13.9Hz), 5.12(1H,d.J=13.9Hz). 6.66(1H,d,J=5.7Hz), 7.30-7.45(3H,m), 7.67-7.76(1H,m), 7.82-7.92(1H,m), 8.38 (1H,d,J=5.7Hz).

### Example 25

### 1-[2-[[[3-Methyl-4-(2,2,2-trifluoroethoxy)-2-pyridinyl]methyl]sulfinyl]-1H-benzimidazol-1-yl]ethyl methoxyacetate

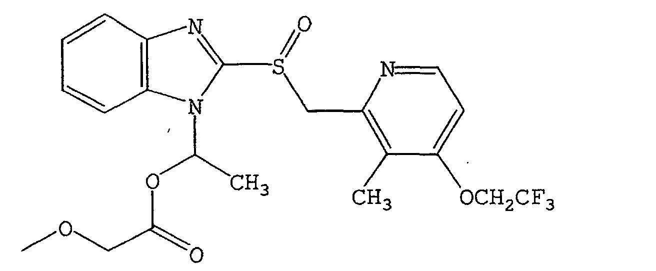

To a mixture of 2-[[[3-methyl-4-(2,2,2-trifluoroethoxy)-2-pyridinyl)methyl]thio]-1H-benzimidazole (2.20 g), sodium hydrogen carbonate (2.62 g) and acetonitrile (60 mL) was added dropwise 1-iodoethyl methoxyacetate (1.83 g). The mixture was stirred at room temperature for 4 days and ethyl acetate (100 mL) was added. The mixture was washed with water (50 mL), aqueous sodium thiosulfate solution (50 mL) and brine (50 mL), dried over anhydrous magnesium sulfate and concentrated under reduced pressure. The residue was purified by silica gel chromatography (eluted with ethyl acetate:hexane=1:1 then 4:1). Fractions containing 1-[2-[[[3-methyl-4-(2,2,2-trifluoroethoxy)-2-pyridinyl]methyl]thio]-1H-benzimidazol-1-yl]ethyl methoxyacetate were collected and concentrated under reduced pressure. To a solution (30 mL) of the residue (1.65 g) in toluene was added 3-chloroperoxybenzoic acid (content: ca. 65%: 0.936 g) by small portions under ice-cooling. After stirring under ice-cooling for 1 hr., ethyl acetate (100 mL) was added and the mixture was washed with aqueous sodium hydrogen carbonate (50 mL), aqueous sodium thiosulfate solution (50 mL) and brine (50 mL). It was dried over anhydrous magnesium sulfate and concentrated under reduced pressure. The residue was purified by silica gel chromatography (eluted with ethyl acetate:hexane=1:1 then successively with ethyl acetate, then successively with ethyl acetate:ethanol=19:1). Crystallization from ethyl acetate-diisopropyl ether and recrystallization from ethyl acetate-diisopropyl ether gave a less polar diastereomer (0.327 g) of the title compound as a colorless solid.
¹H-NMR(CDCl₃): 2.01(3H,d,J=6.3Hz), 2.37(3H,s), 3.36(3H,s), 4.06(2H,s), 4.40(2H,q,J=7.8Hz), 5.08(2H,s), 6.65(1H,d,J=5.6Hz), 7.31-7.45(2H,m), 7.51(1H,q,J=6.3Hz), 7.64-7.73(1H,m), 7.82-7.91(1H,m), 8.35(1H,d,J=5.6Hz).

### Example 26

### 4-[(R)-2-[[[3-Methyl-4-(2,2,2-trifluoroethoxy)-2-pyridinyl]methyl]sulfinyl]-1H-benzimidazol-1-yl]-2,3-dioxolan-2-one

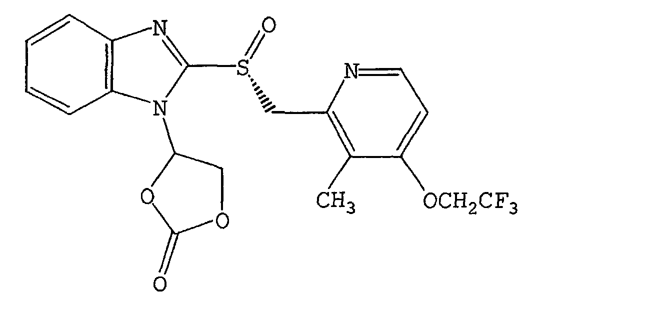

To a solution (150 mL) of (R)-2-[[[3-methyl-4-(2,2,2-trifluproethoxy)-2-pyridinyl]methyl]sulfinyl]-1H-benzimidazole sodium salt (30.0 g) in tetrahydrofuran were added lithium iodide (63.7 g) and 4-(dimethylamino)pyridine (4.69 g) and the mixture was stirred at room temperature for 1 hr. The reaction solution was cooled to -20°C and 4-chloro-1,3-dioxolan-2-one (13.8 mL) was added. The mixture was warmed to 0°C and further stirred for 4 hrs. The reaction solution was added to ethyl acetate (1.5 L) and the mixture was washed successively with 100 mM phosphate buffer, 10% aqueous sodium thiosulfate solution and saturated brine. After drying over anhydrous sodium sulfate, it was concentrated under reduced pressure. The residual solid was washed with diethyl ether and dried under reduced pressure. The obtained solid was recrystallized from acetone to give a less polar diastereomer (18.0 g) of the title compound as a colorless solid.
¹H-NMR (CDCl₃) : 2.20(3H,s), 4.41(2H,q,J=8.0Hz), 4.75-5.00(4H,m), 6.67(1H,d,J=5.8Hz), 7.30-7.50(3H,m), 7.80-7.90(2H,m), 8.23 (1H, d, J=5. 8Hz).

### Example 27

### 4-[2-[[[3-Methyl-4-(2,2,2-trifluoroethoxy)-2-pyridinyl]methyl]sulfinyl]-1H-benzimidazol-1-yl]-1,3-dioxolan-2-one

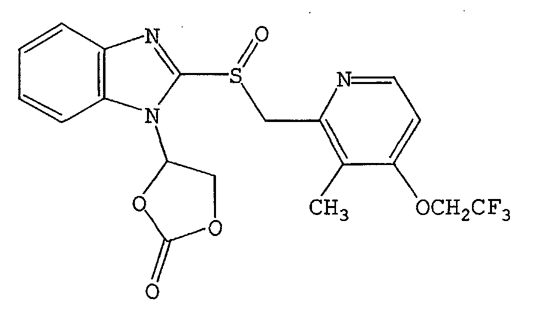

Sodium hydride (736 mg, 60% in oil) was washed with hexane and added to a solution (20 mL) of 2-[[[3-methyl-4-(2,2,2-trifluoroethoxy)-2-pyridinyl]methyl]thio]-1H-benzimidazole (5.0 g) in N,N-dimethylformamide under ice-cooling. The mixture was stirred at 0°C for 1 hr. and chloroethylene carbonate (2.07 g) was added. The mixture was stirred for 1 hr. and extracted with ethyl acetate - water. The organic layer was washed with saturated brine, dried over anhydrous magnesium sulfate and concentrated under reduced pressure. The residue was recrystallized from ethyl acetate to give 4-[2-[[[3-methyl-4-(2,2,2-trifluoroethoxy}-2-pyridinyl]methyl]thio]-2H-benzimidazol-1-yl]-1,3-dioxolan-2-one as a colorless solid (3.0 g). 4-[2-[[[3-Methyl-4-(2,2,2-trifluoroethoxy)-2-pyridinyl]methyl]thio]-1H-benzimidazol-1-yl]-1,3-dioxolan-2-one (1.0 g) was dissolved in methylene chloride (20 mL), and after cooling to 0°C, 3-chloroperoxybenzoic acid (content: ca. 65%, 617 mg) was added. The mixture was stirred for 1 hr. The reaction solution was extracted with methylene chloride - saturated aqueous sodium hydrogen carbonate. The organic layer was washed with saturated brine, dried over anhydrous sodium sulfate and concentrated under reduced pressure. The residue was purified by silica gel chromatography (eluted with ethyl acetate) and recrystallized from ethyl acetate to give a more polar diastereomer (racemate, 400 mg) of the title compound as a colorless solid.
¹H-NMR (CDCl₃) : 2.25(3H,s), 4.35(2H,q,J=7.BHz) , 4.80-5.10(3H,m) , 5.24(1H,d,J=14.6Hz), 6.58(1H,d,J=5.8Hz), 7.30-7.50(3H,m), 7.53(1H,dd,J=4.8,7.6Hz), 7.70-7.80(1H,m), 8.18(1H,d,J=5.8Hz).

### Example 28

### 4-[2-[[[3-Methyl-4-(2,2,2-trifluoroethoxy)-2-pyridinyl]methyl]sulfinyl]-1H-benzimidazol-1-yl]-1,3-dioxolan-2-one

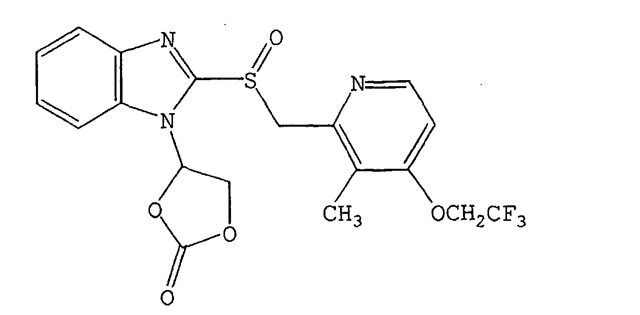

To a solution (50 mL) of 2-[[[3-methyl-4-(2,2,2-trifluoroethoxy)-2-pyridinyl]methyl]sulfinyl]-1H-benzimidazole (2.0 g) in tetrahydrofuran was added sodium hydride (249 mg, 60% in oil) under ice-cooling after washing with hexane. The mixture was stirred at 0°C for 30 min. and lithium iodide (2.05 g) and chloroethylene carbonate (1.38 g) were added. The mixture was stirred for 2 hrs. The reaction solution was extracted with ethyl acetate - water, and the organic layer was washed with saturated brine, dried over anhydrous sodium sulfate and concentrated under reduced pressure. The residue was purified by silica gel column chromatography (eluted with ethyl acetate), and recrystallized from ethyl acetate to give a less polar diastereomer (racemate, 860 mg) of the title compound as a colorless solid.
¹H-NMR (CDCl₃) : 2.19(3H,s), 4.41(2H,q,J=8.0Hz), 4.75-5.00(4H,m), 6.67(1H,d,J=5.8Hz), 7.30-7.50(3H,m), 7.80-7.90(2H,m), 8.23(1H,d,J=5.8Hz).

### Industrial Applicability

The compound of the present invention is converted to lansoprazole, which is a proton pump inhibitor, in living organisms and shows a superior anti-ulcer activity, a gastric acid secretion-suppressive action, a mucosa-protecting action, an *anti-Helicobacter pylori* action and the like. In addition, the compound shows low toxicity and is useful as a pharmaceutical product. Moreover, since the compound is stable to acid, it does not need to be formulated into an enteric-coated preparation, thereby eliminating the cost for formulating enteric-coated preparation. In addition, since the tablet becomes small, it is easy to take for patients with weak swallowing capability, particularly the elderly and children. Furthermore, since absorption is faster than in enteric-coated preparations, expression of a gastric acid secretion-suppressive action is rapid. The preparation is sustainable because it is gradually converted to a conventionally-known proton pump inhibitor in living organisms. Consequently, the compound is useful as anti-ulcer agent and the like.

This application is based on patent application Nos. 292619/2001 and 047204/2002 filed in Japan, the contents of which are all hereby incorporated by reference.

## Claims

1. A benzimidazole compound represented by the formula (I) 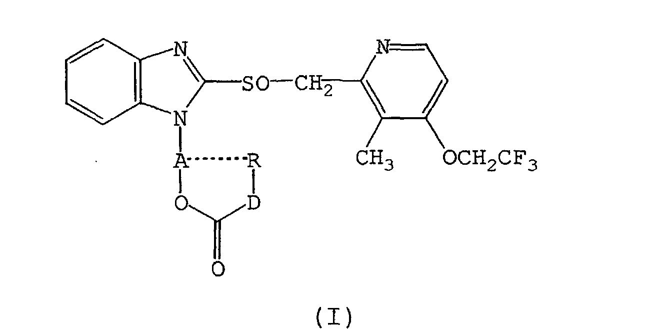 wherein A is an alkylidene group having 2 or more carbon atoms and optionally having substituent(s), R is a hydrocarbon group optionally having substituent(s) or a heterocyclic group optionally having substituent(s), or A and R may be bonded to each other to form a 4- to 8-membered ring optionally having substituent(s), and D is an oxygen atom or a bond, or a salt thereof.

2. The compound of claim 1, wherein (i) A and R are bonded to each other to form a 4- to 8-membered ring optionally having substituent(s) and D is an oxygen atom or a bond, or (ii) A and R are not bonded to each other, A is an alkylidene group having 2 or more carbon atoms and optionally having substituent(s), R is a hydrocarbon group optionally having substituent(s) or a heterocyclic group optionally having substituent(s) and D is an oxygen atom or a bond.

3. The compound of claim 1, which is an (R)-form represented by the formula 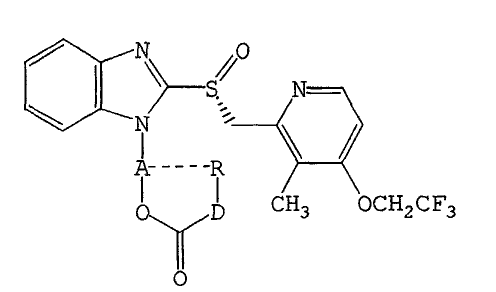 wherein each symbol is as defined in claim 1.

4. The compound of claim 1, wherein R is a C₁₋₆ alkyl group, a C₂₋₆ alkenyl group or a C₂₋₆ alkynyl group, which optionally has substituent(s) selected from the group consisting of (i) a C₆₋₁₄ aryl group, (ii) a hydroxy group, (iii) a halogen, (iv) a C₁₋₆ alkoxy group optionally substituted by halogen, (v) a C₇₋₁₂ aralkyloxy group, (vi) a C₁₋₅ alkoxy-carbonyl group and (vii) an acylamino group, or
a C₃₋₈ cycloalkyl group or a C₆₋₁₄ aryl group, which optionally has substituent(s) selected from the group consisting of (i) a C₆₋₁₄ aryl group, (ii) a hydroxy group, (iii) a halogen, (iv) a C₁₋₆ alkoxy group optionally substituted by halogen, (v) a C₇₋₁₂ aralkyloxy group, (vi) a C₁₋₅ alkoxy-carbonyl group and (vii) a C₁₋₆ alkyl group optionally substituted by halogen, and A is a C₂₋₆ alkylidene group optionally substituted by halogen.

5. The compound of claim 1, wherein R is a C₁₋₆ alkyl group optionally having substituent(s) selected from the group consisting of (i) a C₆₋₁₄ aryl group, (ii) a hydroxy group, (iii) a halogen, (iv) a C₁₋₆ alkoxy group optionally substituted by halogen, (v) a C₇₋₁₂ aralkyloxy group, (vi) a C₁₋₅ alkoxycarbonyl group and (vii) an acylamino group, or
a C₃₋₈ cycloalkyl group or a C₆₋₁₄ aryl group, which optionally has substituent(s) selected from the group consisting of (i) a C₆₋₁₄ aryl group, (ii) a hydroxy group, (iii) a halogen, (iv) a C₁₋₆ alkoxy group optionally substituted by halogen, (v) a C₇₋₁₂ aralkyloxy group, (vi) a C₁₋₅ alkoxy-carbonyl group and (vii) a C₁₋₆ alkyl group optionally substituted by halogen, and A is a C₂₋₆ alkylidene group optionally substituted by halogen.

6. The compound of claim 1, wherein the 4- to 8-membered ring formed by A and R bonded to each other is a ring represented by the formula 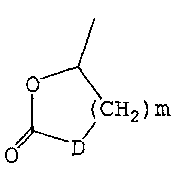 wherein m is an integer of 1 to 3, and other symbols are as defined in claim 1.

7. A benzimidazole compound represented by the formula (I') 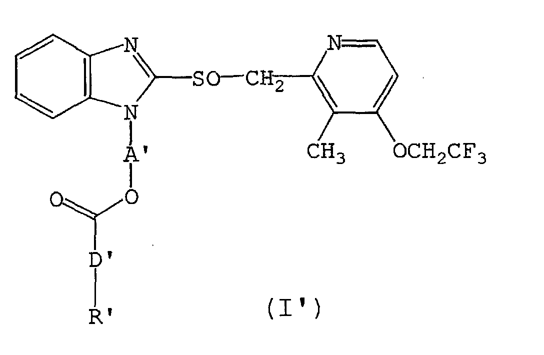 wherein A' is an alkylidene group having 2 or more carbon atoms and optionally having substituent(s), R' is a hydrocarbon group optionally having substituent(s) and D' is an oxygen atom or a bond, or a salt thereof.

8. The compound of claim 7, which is an (R)-form represented by the formula 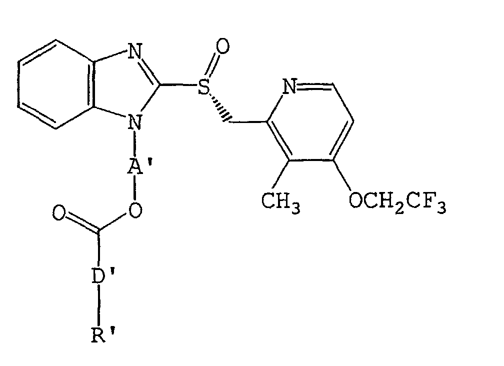 wherein each symbol is as defined in claim 7.

9. The compound of claim 7, wherein R' is a C₁₋₆ alkyl group, a C₂₋₆ alkenyl group or a C₂₋₆ alkynyl group, which optionally has substituent(s) selected from the group consisting of (i) a C₆₋₁₄ aryl group, (ii) a hydroxy group, (iii) a halogen, (iv) a C₁₋₆ alkoxy group optionally substituted by halogen, (v) a C₇₋₁₂ aralkyloxy group and (vi) a C₁₋₅ alkoxy-carbonyl group, or a C₃₋₈ cycloalkyl group or a C₆₋₁₄ aryl group, which optionally has substituent(s) selected from the group consisting of (i) a C₆₋₁₄ aryl group, (ii) a hydroxy group, (iii) a halogen, (iv) a alkoxy group optionally substituted by halogen, (v) a C₇₋₁₂ aralkyloxy group, (vi) a C₁₋₅ alkoxy-carbonyl group and (vii) a C₁₋₆ alkyl group optionally substituted by halogen, and A' is a C₂₋₆ alkylidene group optionally substituted by halogen.

10. The compound of claim 7, wherein R' is a C₁₋₆ alkyl group optionally having substituent(s) selected from the group consisting of (i) a C₆₋₁₄ aryl group, (ii) a hydroxy group, (iii) a halogen, (iv) a C₁₋₆ alkoxy group optionally substituted by halogen, (v) a C₇₋₁₂ aralkyloxy group and (vi) a C₁₋₅ alkoxy-carbonyl group, or
a C₃₋₈ cycloalkyl group or a C₆₋₁₄ aryl group, which optionally has substituent(s) selected from the group consisting of (i) a C₆₋₁₄ aryl group, (ii) a hydroxy group, (iii) a halogen, (iv) a C₁₋₆ alkoxy group optionally substituted by halogen, (v) a C₇₋₁₂ aralkyloxy group, (vi) a C₁₋₅ alkoxy-carbonyl group and (vii) a C₁₋₆ alkyl group optionally substituted by halogen, and A' is a C₂₋₆ alkylidene group optionally substituted by halogen.

11. The compound of claim 7, wherein A' is an ethylidene group or a propylidene group.

12. The compound of claim 7, wherein A' is an ethylidene group.

13. The compound of claim 7, wherein A' is an ethylidene group and R' is a C₁₋₆ alkyl group or a C₃₋₈ cycloalkyl group.

14. A production method of the compound of claim 1 or a salt thereof, which comprises (1) condensing a compound represented by the formula (II) 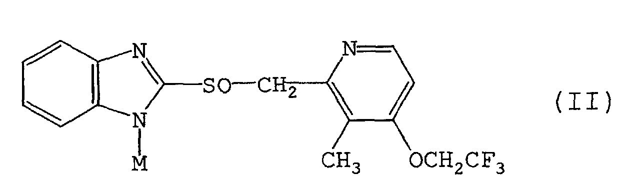 wherein M is a hydrogen atom, a metal cation or a quaternary ammonium ion, or a salt thereof, with a compound represented by the formula (III) 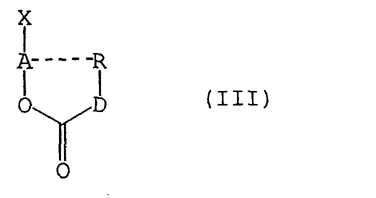 wherein X is a leaving group, A is an alkylidene group having 2 or more carbon atoms and optionally having substituent(s), R is a hydrocarbon group optionally having substituent(s) or a heterocyclic group optionally having substituent(s), or A and R may be bonded to each other to form a 4- to 8-membered ring optionally having substituent(s), and D is an oxygen atom or a bond, or
(2) subjecting a compound represented by the formula (IV) 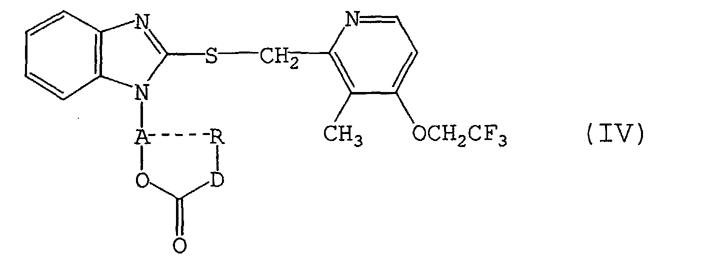 wherein each symbol is as defined above, or a salt thereof, to an oxidation reaction.

15. A pharmaceutical composition comprising the compound of claim 1 or 7.

16. The pharmaceutical composition of claim 15, which is an agent for the prophylaxis or treatment of peptic ulcer, gastritis, reflux esophagitis, symptomatic gastroesophageal reflux disease (symptomatic GERD), NUD, gastric cancer, gastric MALT lymphoma, Zollinger-Ellison syndrome, gastric hyperacidity or upper gastrointestinal hemorrhage.

17. The pharmaceutical composition of claim 15, which is an agent for the eradication of *Helicobacter pylori.*

18. A commercial package comprising the pharmaceutical composition of claim 16 and a written matter associated therewith, the written matter stating that the pharmaceutical composition can or should be used for the prophylaxis or treatment of peptic ulcer, gastritis, reflux esophagitis, symptomatic gastroesophageal reflux disease (symptomatic GERD), NUD, gastric cancer, gastric MALT lymphoma, Zollinger-Ellison syndrome, gastric hyperacidity or upper gastrointestinal hemorrhage.

19. A commercial package comprising the pharmaceutical composition of claim 17 and a written matter associated therewith, the written matter stating that the pharmaceutical composition can or should be used for the eradication of *Helicobacter pylori.*

20. A method for the prophylaxis or treatment of peptic ulcer, gastritis, reflux esophagitis, symptomatic gastroesophageal reflux disease (symptomatic GERD), NUD, gastric cancer, gastric MALT lymphoma, Zollinger-Ellison syndrome, gastric hyperacidity or upper gastrointestinal hemorrhage, which comprises administering the compound of claim 1 or 7.

21. A method for eradicating *Helicobacter pylori,* which comprises administering the compound of claim 1 or 7.

22. Use of the compound of claim 1 or 7 for the production of an agent for the prophylaxis or treatment of peptic ulcer, gastritis, reflux esophagitis, symptomatic gastroesophageal reflux disease (symptomatic GERD), NUD, gastric cancer, gastric MALT lymphoma, Zollinger-Ellison syndrome, gastric hyperacidity or upper gastrointestinal hemorrhage.

23. Use of the compound of claim 1 or 7 for the production of an agent for the eradication of *Helicobacter pylori.*
